(19) Europäisches Patentamt
European Patent Office
Office européen des brevets

(11) EP 4 538 328 A1

(12) **EUROPEAN PATENT APPLICATION**
published in accordance with Art. 153(4) EPC

(43) Date of publication:
**16.04.2025 Bulletin 2025/16**

(21) Application number: **23823923.0**

(22) Date of filing: **13.06.2023**

(51) International Patent Classification (IPC):
***C08L 101/06** (2006.01)* ***A61K 8/02** (2006.01)*
***A61K 8/06** (2006.01)* ***A61K 8/81** (2006.01)*
***A61Q 19/00** (2006.01)* ***C08F 216/06** (2006.01)*
***C08F 257/02** (2006.01)* ***C08F 265/06** (2006.01)*

(52) Cooperative Patent Classification (CPC):
**A61K 8/02; A61K 8/06; A61K 8/81; A61Q 19/00; C08F 216/06; C08F 257/02; C08F 265/06; C08L 101/06**

(86) International application number:
**PCT/JP2023/021935**

(87) International publication number:
**WO 2023/243633 (21.12.2023 Gazette 2023/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.06.2022 JP 2022095286**

(71) Applicant: **KIRIN HOLDINGS KABUSHIKI KAISHA**
**Nakano-ku, Tokyo 164-0001 (JP)**

(72) Inventors:
- **YAMAZAKI, Takashi**
**Tokyo 164-0001 (JP)**
- **OGAWA, Akiko**
**Tokyo 164-0001 (JP)**
- **TSUJI, Toshikazu**
**Tokyo 164-0001 (JP)**

(74) Representative: **ABG Intellectual Property Law, S.L.**
**Avenida de Burgos, 16D**
**Edificio Euromor**
**28036 Madrid (ES)**

(54) **PARTICLES USED FOR FORMATION OF PICKERING EMULSION OR LIKE**

(57) A particle containing a hydrophobic segment that contains a hydrophobic monomer unit and has a cationic group, and a hydrophilic segment that contains at least a hydrophilic monomer unit represented by Formula (I) is disclosed. The use of this particle enables the formation of a stable emulsion composition without using a surfactant, and by adopting the present disclosure, it is possible to provide particles with high versatility. This particle can be used to form a Pickering emulsion and can be used with versatility particularly in the fields of cosmetics, pharmaceuticals, and other products, which are brought into direct contact with the human body.

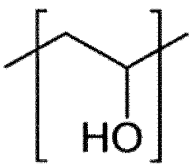

(I)

FIG. 2

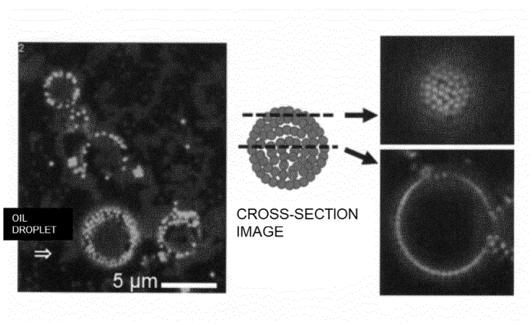


EP 4 538 328 A1

## Description

CROSS-REFERENCE TO RELATED APPLICATION

This patent application claims priority based on Japanese Patent Application No. 2022 095286 (filing date: June 13, 2022) which is a patent application filed earlier in Japan. The entire disclosure in this prior patent application is incorporated herein by reference.

TECHNICAL FIELD

The present invention relates to a particle containing a predetermined hydrophobic monomer unit and a predetermined hydrophilic monomer unit, and more preferably relates to the particle used to form a Pickering emulsion.

BACKGROUND ART

As for an emulsion composition (emulsion) such as an oil-in-water type composition or a water-in-oil type composition, the emulsion composition is generally stable because an aqueous component and an oily component are finely mixed through the emulsification of a surfactant blended. Such an emulsion composition is widely used in daily necessities, personal care products, and biological application fields. However, in a case where a large amount of a surfactant is used in an emulsion composition, problems such as stickiness and irritation can occur when the emulsion composition is directly applied to a human body such as skin, and there are quite a few consumers who are concerned about the influence on a human body of a surfactant that can be absorbed into the human body. Therefore, it is desired to reduce the amount of the surfactant used in the emulsion composition as much as possible.

Under such a circumstance, attempts have been made to use additives with emulsifying properties other than the surfactant in the emulsion composition. As one of such attempts, attention has been focused on a Pickering emulsion which is an emulsion composition emulsified by allowing solid particles to be present near an interface between an oil phase and an aqueous phase.

Fig. 1 is a diagram schematically comparing an emulsion with a surfactant and a Pickering emulsion. Although the present invention is not bound by any theory, it is presumed that a Pickering emulsion is usually in a state where mineral particles or amphiphilic organic particles are present at the interface of an aqueous or oily droplet, thereby stabilizing the structure of the droplet. As an example of a Pickering emulsion, for example, a Pickering emulsion containing a powder that is adsorbed onto an emulsification interface, and an acrylic polymer is disclosed in Patent Document 1. According to the invention described in Patent Document 1, it is possible to provide a Pickering emulsion having excellent emulsion stability.

In addition, it is disclosed in Patent Document 2 that a cement composition contains a cement, an acrylic copolymer redispersible resin powder, an expansive additive, and a shrinkage reducing agent, in which in the acrylic copolymer redispersible resin powder, primary particles of the resin powder have an average particle diameter of 0.2 μm or more and 0.8 μm or less, and surfaces of the primary particles of the resin powder are covered with a water-soluble protective colloid of polyvinyl alcohol (PVA). The cement composition described in Patent Document 2 can be kneaded with water to obtain a mortar that is excellent in workability such as trowel plastering, has a small curing shrinkage, and has high durability of adhesion.

PRIOR ART REFERENCES

Patent Documents

Patent Document 1: JP 2019-202962 A
Patent Document 2: JP 2009-102216 A

SUMMARY OF THE INVENTION

However, conventionally, various particles used to form an emulsion composition such as a Pickering emulsion have had insufficient emulsification and a lack of versatility depending on the properties of constituent parts of the particles. In particular, when particles were used without a relatively large amount of a surfactant in combination, an emulsion composition formed of the particles tended to be unstable. In addition, there was a problem in that inorganic particles used to form a Pickering emulsion tend to have low emulsion stability, whereas organic particles tend to have insufficient emulsification and low versatility due to the characteristics of monomers used.

Therefore, the object of the present invention is to provide a particle with high versatility, which enables the formation of a stable emulsion composition without using a surfactant.

The inventors of the present invention conducted intensive studies in view of the above problems. As a result, the present inventors have found that the above problems can be solved by using a particle that has a hydrophobic segment containing a hydrophobic monomer unit and having a cationic group, and a hydrophilic segment containing a predetermined hydrophilic monomer unit, and have completed the present invention.

That is, the present invention provides the following aspects.

A first aspect of the present invention is a particle including a hydrophobic segment that contains a hydrophobic monomer unit and has a cationic group, and a hydrophilic segment that contains at least a hydrophilic monomer unit represented by Formula (I) below.

[Chem. 1]

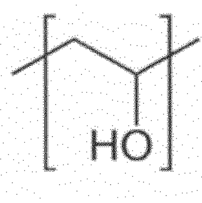

(I)

A second aspect of the present invention is the particle according to the first aspect, wherein a part or entirety of a surface of the hydrophobic segment is directly or indirectly covered with the hydrophilic monomer or a polymer of the hydrophilic monomer.

A third aspect of the present invention is the particle according to the first or second aspect, wherein the cationic group is derived from a cationic radical polymerization initiator.

A fourth aspect of the present invention is the particle according to any one of the first to third aspects, wherein the hydrophobic monomer unit is a monomer unit having an ethylenically unsaturated double bond.

A fifth aspect of the present invention is the particle according to the fourth aspect, wherein the monomer unit having an ethylenically unsaturated double bond is a (meth)acrylic acid monomer unit.

A sixth aspect of the present invention is the particle according to the fourth aspect, wherein the monomer unit having an ethylenically unsaturated double bond is a styrene monomer unit.

A seventh aspect of the present invention is the particle according to any one of the first to sixth aspects, wherein the cationic radical polymerization initiator is at least one selected from the group consisting of 2,2'-[diazene-1,2-diylbis(propane-2,2-diyl)]bis(1,3-dimethyl-4,5-dihydro-1H-imidazole-3-ium)=ditrifluoromethanesulfonate (ADIP), 2,2'-[diazene-1,2-diylbis(propane-2,2-diyl)]bis(1,3-dimethyl-4,5-dihydro-1H-imidazole-3-ium)=dichloride (ADIP-Cl), 2,2'-azobis(2-methylpropionamidine)dihydrochloride (V-50), 2,2'-azobis[2-(2-imidazolin-2-yl)propane]dihydrochloride (VA-044), and 2,2'-azobis[2-(2-imidazoline-2-yl)propane] (VA-061).

An eighth aspect of the present invention is the particle according to any one of the first to seventh aspects, wherein the hydrophilic segment further contains a monomer unit represented by Formula (II) below, and a ratio of a number of the monomer unit represented by Formula (I) to a total of the number of the monomer unit represented by Formula (I) and a number of the monomer unit represented by Formula (II) (degree of saponification) is 70% or more and 99% or less.

[Chem. 2]

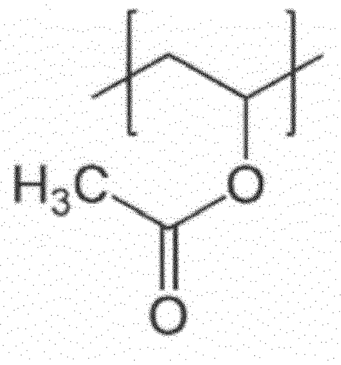

(II)

A ninth aspect of the present invention is the particle according to the eighth aspect, wherein a ratio of the number of the monomer unit represented by Formula (I) to a total of the number of the monomer unit represented by Formula (I) and the number of the monomer unit represented by Formula (II) (degree of saponification) is 78% or more and 90% or less.

A tenth aspect of the present invention is the particle according to any one of the first to ninth aspects, wherein a mass corresponding to the hydrophilic segment is equivalent to 1 part by mass or more and 6 parts by mass or less with respect to 100 parts by mass of a mass corresponding to the hydrophobic segment.

An eleventh aspect of the present invention is the particle according to any one of the first to tenth aspects, wherein the particle has an average zeta potential of +10 mV or more and +60 mV or less.

A twelfth aspect of the present invention is the particle according to any one of the first to eleventh aspects, wherein the particle has a cumulant average diameter of 100 nm or more and 500 nm or less.

A thirteenth aspect of the present invention is the particle according to the second aspect, wherein the hydrophobic monomer unit and the hydrophilic monomer unit are bonded by a covalent bond, and the part or entirety of the surface of the hydrophobic segment is directly covered with the hydrophilic monomer or a polymer of the hydrophilic monomer.

A fourteenth aspect of the present invention is the particle according to the second aspect, wherein the hydrophobic monomer unit and the hydrophilic monomer unit are bonded by an intermolecular force, and the part or entirety of the surface of the hydrophobic segment is indirectly covered with the hydrophilic monomer or a polymer of the hydrophilic monomer.

A fifteenth aspect of the present invention is the particle according to the fourteenth aspect, wherein a bonding by the intermolecular force is induced by heat treatment.

A sixteenth aspect of the present invention is the particle according to any one of the first to fifteenth aspects, wherein a difference ($T_{0.01}$ - $T_1$) between an interfacial tension value ($T_{0.01}$) and an interfacial tension value ($T_1$) is 10 mN/m or more, the interfacial tension value ($T_{0.01}$) being between an aqueous dispersion containing 0.01% by mass of the particle and decamethylcyclopentasiloxane, and the interfacial tension value ($T_1$) being between an aqueous dispersion containing 1% by mass of the particle and decamethylcyclopentasiloxane.

A seventeenth aspect of the present invention is the particle according to the sixteenth aspect, wherein the interfacial tension value ($T_{0.01}$) is 25 mN/m or less.

An eighteenth aspect of the present invention is the particle according to any one of the first to seventeenth aspects, wherein a relative ratio between a molecular weight of the hydrophobic segment alone and a molecular weight of the hydrophilic segment alone per particle unit is such that the hydrophilic segment corresponds to 1 or more and 6 or less when the hydrophobic segment corresponds to 100.

A nineteenth aspect of the present invention is the particle according to any one of the first to eighteenth aspects, wherein a residual ratio of an insoluble fraction after extraction with a Soxhlet extractor using chloroform as an extraction solvent is 5% or more.

A twentieth aspect of the present invention is the particle according to any one of the first to nineteenth aspects, wherein the particle is used to form a Pickering emulsion.

A twenty-first aspect of the present invention is a method for producing the particle according to any one of the first to twentieth aspects, the method including the steps of: polymerizing a hydrophobic monomer in a presence of a cationic radical polymerization initiator and an emulsifier; polymerizing a hydrophilic monomer separately from the hydrophobic monomer or after completion of polymerizing the hydrophobic monomer; and bonding or associating the hydrophobic monomer polymerized and the hydrophilic monomer polymerized.

A twenty-second aspect of the present invention is the particle according to any one of the first to twentieth aspects, wherein the particle contains a polymer having a hydrophobic segment and a hydrophilic segment.

A twenty-third aspect of the present invention is the particle according to the twenty-second aspect, wherein the polymer having a hydrophobic segment and a hydrophilic segment has a number-average molecular weight of 1,000 or more and 500,000 or less.

A twenty-fourth aspect of the present invention is the particle according to the twenty-second or twenty-third aspect, wherein the polymer having a hydrophobic segment and a hydrophilic segment has a glass transition temperature of 30°C or higher and 200°C or lower.

A twenty-fifth aspect of the present invention is the particle according to any one of the first to twentieth aspects or the twenty-second to twenty-fourth aspects, wherein the hydrophobic monomer is at least one selected from the group consisting of methyl methacrylate, styrene, and butyl acrylate.

A twenty-sixth aspect of the present invention is the particle according to any one of the twentieth aspect or the twenty-second to twenty-fifth aspects, wherein the Pickering emulsion is for an application to a skin.

A twenty-seventh aspect of the present invention is the particle according to any one of the twentieth aspect or the twenty-second to twenty-sixth aspects, wherein the Pickering emulsion contains an aromatic compound.

A twenty-eighth aspect of the present invention is the particle according to the twenty-seventh aspect, wherein the aromatic compound is an ultraviolet absorbing agent or a preservative.

A twenty-ninth aspect of the present invention is the particle according to any one of the twentieth aspect or the twenty-second to twenty-eighth aspects, wherein the Pickering emulsion is a Pickering emulsion that prevents the aromatic compounds contained in the Pickering emulsion from penetrating a skin.

A thirtieth aspect of the present invention is the particle according to the thirteenth aspect, wherein the hydrophobic monomer unit and the hydrophilic monomer unit are bonded by a covalent bond, and the part or entirety of the surface of the hydrophobic segment is directly covered with a polymer of the hydrophilic monomer, and the polymer of the hydrophilic monomer constitutes a layer having a thickness of 0.001 nm to 200 nm.

According to the present invention, it is possible to provide the particle with high versatility, which enables the formation of the stable emulsion composition without using the surfactant. The particle of the present invention can be used to form the Pickering emulsion, and can be used with versatility particularly in the fields of cosmetics, pharmaceuticals, and other products, which are brought into direct contact with the human body.

BRIEF DESCRIPTION OF THE DRAWINGS

Fig. 1 is a diagram schematically comparing an emulsion with a surfactant and a Pickering emulsion.
Fig. 2 is microscopic images of emulsion compositions prepared in Examples 1 to 4 upon a fluorescence observation with a 100 times oil immersion objective lens.
Fig. 3 is a diagram illustrating the results of the interfacial tension measurement performed on dispersions of Production Example 1 and Production Example 4 by the pendant drop method.
Fig. 4 is a diagram illustrating the results of analyzing dry powders obtained by washing, concentrating, freezing, and drying the dispersions of Production Example 1 and Production Example 4 by Fourier transform infrared spectroscopy (FT-IR).
Fig. 5 is a diagram illustrating the results of analyzing dry powders obtained by washing, concentrating, freezing, and drying the dispersions of Production Example 1 and Production Example 4 by Fourier transform infrared spectroscopy (FT-IR).
Fig. 6 is a diagram illustrating the results of analyzing dry powders obtained by washing, concentrating, freezing, and drying the dispersions of Production Example 1 and Production Example 4 by Fourier transform infrared spectroscopy (FT-IR).

MODES FOR CARRYING OUT THE INVENTION

Hereinafter, the embodiments of the present invention will be described in detail with reference to the drawings. Note that, the following embodiments are described for illustrative purposes, and the following description of the present embodiments should not be construed as limiting the scope of the claims.

<Particle>

A particle of the present embodiment, which is an example of the particle of the present invention, has a hydrophobic segment containing a hydrophobic monomer unit and having a cationic group, and a hydrophilic segment containing a predetermined hydrophilic monomer unit. The particle of the present embodiment is preferably used for the formation of a Pickering emulsion. The particle of the present embodiment enables the formation of a stable emulsion composition without a surfactant while having storage stability. In addition, the particle of the present embodiment has high versatility and can also be used for the formation of a Pickering emulsion.

Furthermore, the particle of the present embodiment provides a good texture, and can be widely used particularly in the fields of cosmetics, pharmaceuticals, and other products, which are brought into direct contact with the human body. Such an emulsion composition formed by using the particle of the present embodiment can be prepared without using an apparatus that industrially generates a strong external force, such as an ultrasonic treatment apparatus or a homogenizer, and does not necessarily require heat treatment for stabilizing a dispersion state. Therefore, the use of the particle of the present embodiment can also contribute to the reduction of the environmental load during the adjustment of the emulsion composition.

[Hydrophobic Segment]

As described above, the hydrophobic segment contains a hydrophobic monomer unit, and can take the form of a polymer obtained by polymerizing such a hydrophobic monomer unit as a main constituent monomer. Such a polymer may be obtained by either monopolymerization or copolymerization as long as it is within the range where the effects of the

present invention are achieved. Examples of the "hydrophobic monomer unit" can include a monomer unit obtained from a monomer having an ethylenically unsaturated double bond. Here, examples of the monomer having an ethylenically unsaturated double bond can include a polymerizable monomer having a carbon-carbon double bond in the molecule, and more specifically, a (meth)acrylic acid monomer unit, a styrene monomer unit, and other monomer units can be exemplified.

Here, examples of the (meth)acrylic acid monomer unit can include a monomer unit derived from alkyl (meth) acrylate having an alkyl ester group having 1 or more and 6 or less carbon atoms. Here, examples of the alkyl ester group having 1 or more and 6 or less carbon atoms can include a methyl ester group, an ethyl ester group, an n-propyl ester group, an n-butyl ester group, an n-pentyl ester group, and an n-hexyl ester group. In the alkyl ester group, one or more hydrogen atoms of the alkyl group constituting the alkyl ester group may be substituted with a hydroxyl group as necessary. As the alkyl ester group, an alkyl ester group having 1 or more and 4 or less carbon atoms is more preferable from the viewpoint of ensuring the sufficient storage stability of the particle.

As such alkyl (meth)acrylate, methyl methacrylate (MMA), butyl methacrylate (BMA), or 2-hydroxyethyl methacrylate (HEMA) is preferably used, and methyl methacrylate is more preferably used. In a case where these monomers are used, the storage stability of the resulting particle is improved, and the emulsifying ability of the particle is further excellent.

In a case where the (meth)acrylic acid monomer unit has an acidic group such as a carboxyl group, the carboxyl group may be in the form of a salt, for example, a sodium salt or potassium salt.

Examples of the styrene monomer unit can include styrene, α-methylstyrene, o-methylstyrene, m-methylstyrene, p-methylstyrene, ethylstyrene, isobutylstyrene, t-butylstyrene, o-bromostyrene, m-bromostyrene, p-bromostyrene, o-chlorostyrene, m-chlorostyrene, p-chlorostyrene, and other styrene monomer units. Among these, styrene is preferably used from the viewpoint of ensuring sufficient storage stability and emulsifying ability of the resulting particle.

In a case where a hydrophobic segment containing a hydrophobic monomer unit and having a cationic group is prepared by using the monomers as described above, the monomers may be usually radically polymerized.

[Cationic Group]

The cationic group contained in the hydrophobic segment of the particle of the present embodiment is generally assumed to be a cationic group derived from a cationic radical polymerization initiator, but is not limited to merely the cationic group derived from the cationic radical polymerization initiator, and examples thereof can include a cationic group derived from any compound or polymerization unit capable of introducing any cationic group to the end of the hydrophobic segment or into the hydrophobic segment. Specific examples of such a cationic group can include a primary amino group, a secondary amino group, a tertiary amino group, a quaternary ammonium group, an imidazole group, an imidazolium group, a pyridyl group, a pyridinium group, a piperidyl group, a piperidinium group, a pyrrolidinyl group, a pyrrolidinium group, a phosphonium group, and other cationic groups. However, in the present embodiment, it is particularly preferable to employ, as the cationic group, a cationic group introduced to the end of the hydrophobic segment by the polymerization of hydrophobic monomers using the cationic radical polymerization initiator as a polymerization initiator. By using the cationic radical polymerization initiator, an end structure derived from the cationic radical polymerization initiator can be covalently bonded to the monomer unit at the end of the hydrophobic segment. In the present embodiment, a core part contains the hydrophobic segment, and the cationic group is disposed on a surface of the core part or in the vicinity thereof, so that the particle can have excellent emulsifying ability.

(Cationic Radical Polymerization Initiator)

As the cationic radical polymerization initiator that can be used in the present embodiment, those having both safety after polymerization and reactivity as a radical polymerization initiator can be generally used. More specifically, it is preferable to use one or more selected from the group consisting of 2,2'-[diazene-1,2-diylbis(propane-2,2-diyl)]bis(1,3-dimethyl-4,5-dihydro-1H-imidazole-3-ium)=ditrifluoromethanesulfonate (ADIP), 2,2'-[diazene-1,2-diylbis(propane-2,2-diyl)]bis(1,3-dimethyl-4,5-dihydro-1H-imidazole-3-ium)=dichloride (ADIP-Cl), 2,2'-azobis(2-methylpropionamidine)dihydrochloride (V-50, manufactured by FUJIFILM Wako Pure Chemical Corporation), 2,2'-azobis[2-(2-imidazolin-2-yl) propane]dihydrochloride (VA-044, manufactured by FUJIFILM Wako Pure Chemical Corporation), and 2,2'-azobis [2-(2-imidazoline-2-yl)propane] (VA-061, manufactured by FUJIFILM Wako Pure Chemical Corporation). By using these cationic radical polymerization initiators, the emulsifying ability of the resulting particle is further improved.

Examples of the radical polymerization initiator that can be used in the present embodiment can include a cationic radical polymerization initiator represented by General Formula (III). For further details of the following cationic radical polymerization initiator, see, for example, JP 2017-051113 A. The contents of the document are incorporated herein by reference. Specific examples of the cationic radical polymerization initiator represented by General Formula (III) can include ADIP and ADIP-Cl described above. By using ADIP or ADIP-Cl, the particle of the present embodiment can be prepared under milder reaction conditions, and the deterioration of storage stability of the particle due to damage such as

heating during the preparation of the particle can also be minimized. In addition, the particle of the present embodiment can be prepared under milder reaction conditions by using ADIP or ADIP-Cl as described above, which can also contribute to reducing environmental impact during the synthesis.

[Chem. 3]

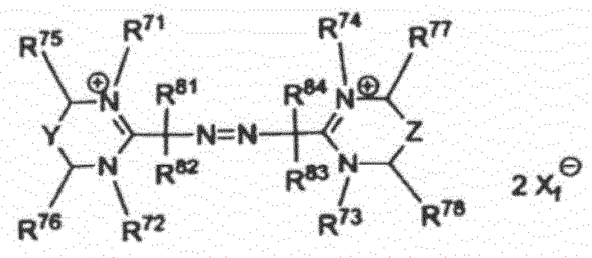

(III)

In the formula,

Y represents a single bond or $CR^{85}$,
Z represents a single bond or $CR^{86}$,
$R^{72}$, $R^{73}$, $R^{75}$, $R^{76}$, $R^{77}$, $R^{78}$, $R^{85}$, and $R^{86}$ are each independently selected from the group consisting of a hydrogen atom, $C_{1-6}$ alkyl, $C_{1-6}$ alkoxy, $C_{1-6}$ alkylcarbonyl, phenyl, and hydroxy, where the $C_{1-6}$ alkyl, $C_{1-6}$ alkoxy, $C_{1-6}$ alkylcarbonyl, and phenyl may be further substituted with 1 or 2 substituents selected from the group consisting of $C_{1-6}$ alkyl, $C_{1-6}$ alkoxy, $C_{1-6}$ alkylcarbonyl, phenyl, and hydroxy;
$R^{72}$ and $R^{73}$ each independently may further represent adamantyl or $C_{1-6}$ alkyl substituted with $Si(OCH_3)_2(CH_3)$, or $R^{75}$ and $R^{76}$, or $R^{77}$ and $R^{78}$ may form $-(CH_2)_{3-5}-$ together;
$R^{81}$, $R^{82}$, $R^{83}$, and $R^{84}$ are substituents selected from the group consisting of $C_{1-4}$ alkyl, $C_{1-4}$ alkylcarbonyl, and $C_{1-3}$ alkoxy, where the $C_{1-4}$ alkyl may be substituted with one $C_{1-3}$ alkoxy group; and
$R^{71}$ and $R^{74}$ are each independently a $C_{1-3}$ alkyl group, and $X_f^-$ is a counter anion.

The "counter anion" in the cationic radical polymerization initiator of Formula (III) is not particularly limited as long as it is an anion usually used as a counter anion of an organic compound in the technical field of organic chemistry, and examples thereof include halide anions (chloride ions, bromide ions, fluoride ions, and iodide ions), conjugate bases of organic acids (for example, acetate ions, citrate ions, and trifluoroacetate ions), nitrate ions, sulfate ions, carbonate ions, and other counter anions. Preferable examples of the counter anion in the present embodiment include trifluoromethanesulfonate ions (triflate), chloride ions, nitrate ions, and other counter anions. Among these, chloride ions, acetate ions, and citrate ions are preferable, and chloride ions are more preferable from the viewpoint of being able to significantly improve the storage stability of a dispersion containing the particle of the present embodiment, increasing the yield of the particle in the preparation of the particle of the present embodiment, and reducing production costs.

[Hydrophilic Segment]

The hydrophilic segment contained in the particle of the present embodiment contains, more specifically, a hydrophilic monomer unit represented by Formula (I), and can take the form of a polymer obtained by polymerizing such a hydrophilic segment unit as a main constituent monomer. Such a polymer may be obtained by either monopolymerization or copolymerization as long as it is within the range where the effects of the present invention are achieved.

[Chem. 4]

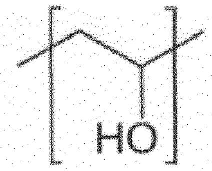

(I)

The hydrophilic monomer unit represented by Formula (I) is not particularly limited but can be generally obtained by polymerizing vinyl acetate through a radical polymerization reaction to saponify acetate ester group. Therefore, in the present embodiment, although the hydrophilic segment always has the hydrophilic monomer unit represented by Formula (I), not all monomer units of the hydrophilic segment are limited to those represented by Formula (I). More specifically, the hydrophilic segment can contain a monomer unit represented by the formula (II), which is derived from vinyl acetate, and a monomer unit represented by the formula (I) and the upper limit of a ratio of hydroxyl groups to the total number of acetic acid groups in the monomer units represented by Formula (II) and hydroxyl groups in the monomer units represented by Formula (I) (in the present invention, may be simply referred to as a "degree of saponification") can be set to 99%, 98%, or 96%, and the lower limit thereof can be set to 70%, 75%, or 78%, from the viewpoint of enhancing the storage stability and emulsifying ability of the particle itself and improving the storage stability of the emulsion composition formed by using the particle. In addition, the "degree of saponification" is preferably 70% or more and 99% or less, more preferably 75% or more and 98% or less, and still more preferably 78% or more and 96% or less. This "degree of saponification" is adjusted to adjust a degree of hydrophilicity in the hydrophilic segment of the particle of the present embodiment. Thus, it is preferable to appropriately adjust the "degree of saponification" according to the intended use of the particle of the present embodiment.

[Chem. 5]

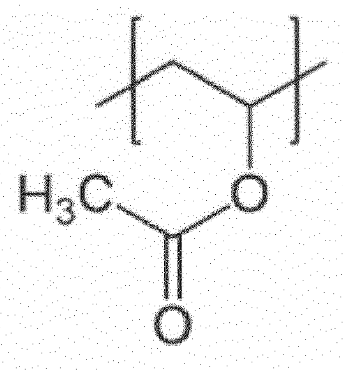

(II)

[Core Part of Particle]

The particle of the present embodiment has an organic group, which is exposed at least on a surface of the particle, and a core part thereof may be composed of an organic group alone, but is not limited to being composed of such an organic group alone, and may contain an inorganic material. In a case where the core part of the particle of the present embodiment contains an inorganic material, it can be mentioned to bond or associate a polymer containing a hydrophobic monomer unit to the surface of the inorganic substance in the core part.

[Crosslinking Monomer]

As an organic monomer unit constituting the core part, a crosslinking monomer unit may be used, and in such an aspect, a crosslinking monomer may be partially used as one of the monomers for preparing the particle of the present embodiment. Specific examples of such a crosslinking monomer can include a monomer containing two or more ethylenically unsaturated double bonds in the molecule, which is usually used as a crosslinking agent. However, in the present embodiment, such a crosslinking monomer may not be used. In a case where the crosslinking monomer is used, a content thereof usually can be set with the upper limit of 20%, 10%, or 5%, the lower limit of 0.1% or 3%, and within a range of 0.1% or more and 20% or less, preferably 3% or more and 10% or less, and more preferably 5% or less, in terms of

moles with respect to the monomer constituting the hydrophobic segment and the monomer constituting the hydrophilic segment, respectively, from the viewpoint of ensuring the sufficient storage stability of the particle. Specific examples of such a crosslinking monomer can include N,N'-methylenebisacrylamide, N,N'-ethylenebisacrylamide, N,N'-methylenebismethacrylamide, N,N'-ethylenebismethacrylamide, ethylene glycol diacrylate, ethylene glycol dimethacrylate, polyethylene glycol diacrylate, polyethylene glycol dimethacrylate, divinylbenzene, and other crosslinking monomers.

[Aspect of Combination of Hydrophobic Segment and Hydrophilic Segment]

In the particle of the present embodiment, it is preferable that the surface of the core part of the particle is hydrophobic, and a part or entirety of the surface is directly or indirectly covered with the hydrophilic monomer or a polymer thereof from the viewpoint of further improving the emulsifying ability by the particle. More specifically, the hydrophobic monomer unit and the hydrophilic monomer unit may be bonded by a covalent bond (aspect of being "directly covered"), or the hydrophobic monomer unit and the hydrophilic monomer unit may be bonded by an intermolecular force (aspect of being "indirectly covered").

In addition, in a case where the hydrophobic monomer unit and the hydrophilic monomer unit are bonded by a covalent bond, a polymer in which the hydrophobic monomer unit and the hydrophilic monomer unit are covalently bonded may be a block copolymer or a graft copolymer; in particular, in a case where the polymer is a graft copolymer, the storage stability of the particle is further improved, so that the polymer in which the hydrophobic monomer unit and the hydrophilic monomer unit are covalently bonded is more preferably a graft copolymer.

Furthermore, in a case where the hydrophobic monomer unit and the hydrophilic monomer unit are bonded by an intermolecular force, the bonding by the intermolecular force is preferably induced by heat treatment from the viewpoint of further improving the storage stability of the particle and the emulsifying ability by the particle. As the heat treatment in this case, for example, a heat treatment at a temperature of 20°C or higher and 100°C or lower, more specifically, 30°C or higher and 80°C or lower for 30 minutes or longer and 480 minutes or shorter can be exemplified.

Additionally, the particle of the present embodiment is not limited to the particle in which the hydrophobic monomer unit and the hydrophilic monomer unit are bonded by a covalent bond alone or the particle in which the hydrophobic monomer unit and the hydrophilic monomer unit are bonded by an intermolecular force alone, and some hydrophobic monomer units may be bonded to hydrophilic monomer units by a covalent bond, and the remaining hydrophobic monomer units may be bonded to hydrophilic monomer units by an intermolecular force. The presence proportion of the hydrophobic monomer unit bonded to the hydrophilic monomer unit by a covalent bond with respect to the total hydrophobic monomer units bonded to the hydrophilic monomer units may have an upper limit of 90%, 70%, or 60%, and a lower limit of 10%, 30%, or 40%, and is preferably 10% or more and 90% or less, more preferably 30% or more and 70% or less, and still more preferably 40% or more and 60% or less, from the viewpoint of improving the emulsifying ability by the particle.

In the aspect described above, from the viewpoint of improving the storage stability of the particle and the emulsifying ability by the particle, the presence proportion of the hydrophobic segment and the hydrophilic segment can be set, with respect to 100 parts by mass of a mass corresponding to the hydrophobic segment, such that a mass corresponding to the hydrophilic segment has an upper limit of 6 parts by mass or 4 parts by mass, and a lower limit of 1 part by mass or 3 parts by mass, and is preferably equivalent to 1 part by mass or more and 6 parts by mass or less, and more preferably 3 parts by mass or more and 4 parts by mass or less.

[Molecular Weight]

In the particle of the present embodiment, the molecular weight of a polymer (a polymer having a hydrophobic segment and a hydrophilic segment) when a hydrophobic segment in the form of a polymer and a hydrophilic segment in the form of a polymer are bonded by a covalent bond, or single molecule, or the total the molecular weight of hydrophobic and hydrophilic single molecules when they are bonded by a non-covalent bond, is not particularly limited, but can be calculated, for example, by dissolving the particles in an organic solvent such as chloroform and measuring the number-average molecular weight in gel permeation chromatography using polystyrene as the standard. In this case, a separating operation of the hydrophobic segment and the hydrophilic segment may be performed as necessary in a case where the hydrophobic segment in the form of a polymer and the hydrophilic segment in the form of a polymer are non-covalently bonded. The upper limit of the number-average molecular weight of the polymer having a hydrophobic segment and a hydrophilic segment can be set to 500,000, 30,000, 20,000, or 10,000, and the lower limit thereof can be set to 1,000, 10,000, or 50,000 from the viewpoint of improving the storage stability of the particle. The number-average molecular weight of the polymer having a hydrophobic segment and a hydrophilic segment is preferably 1,000 or more and 500,000 or less, 1,000 or more and 100,000 or less, 5,000 or more and 20,000 or less, 10,000 or more and 500,000 or less, 50,000 or more and 30,000 or less, or 50,000 or more and 30,000 from the viewpoint of improving the storage stability of the particle. In addition, the molecular weight distribution, which is a ratio of the number-average molecular weight and the weight-average molecular weight, is preferably within a range of 1.05 or more and 5 or less, and more preferably within a range of 1.05 or more and 1.7 or less. Furthermore, in the present embodiment, a relative ratio between a molecular weight of the hydrophobic segment alone and a molecular weight of the hydrophilic segment alone per particle unit is preferably such that the hydrophilic segment corresponds to 1 or more and 6 or less when the hydrophobic segment corresponds to 100. By setting the relative ratio within the above-described range, the emulsifying ability by the particle of the present embodiment can be enhanced, and the storage stability of the particle of the present embodiment can be improved.

[Glass Transition Temperature]

The glass transition temperature of the polymer having a hydrophobic segment and a hydrophilic segment can have an upper limit of 200°C, 150°C, or 100°C, and a lower limit of 30°C, 40°C, or 45°C from the viewpoint of enhancing the storage stability of the emulsion composition.

The glass transition temperature of the polymer can be measured by using a differential scanning calorimetry (DSC) method. A specific measurement method of the DSC method conforms to the method described in JIS K 7121 (1987).

The glass transition temperature of the polymer can be, for example, 100°C for polystyrene, 105°C for polymethyl methacrylate, 83°C for polyisopropyl methacrylate, 107°C for polycyclohexyl methacrylate, 110°C for polyphenyl methacrylate, 101°C for polyacrylic acid, 121°C for poly-N,N-dimethylacrylamide, 100°C for polydimethyl itaconate, and 100°C for polydimethyl fumarate.

[Pickering Emulsion]

The Pickering emulsion is preferably for application to skin from the viewpoint of texture and safety.

In the present embodiment, the Pickering emulsion preferably contains an aromatic compound. This is because the risk regarding safety to the human body can be reduced while the stability of the emulsion is maintained.

The aromatic compound includes a group of cyclic unsaturated organic compounds such as benzene, and includes an aromatic hydrocarbon composed of hydrocarbon alone and also a heteroaromatic compound containing an element other than carbon in a ring structure.

The Pickering emulsion is preferably a Pickering emulsion that prevents the aromatic compounds contained in the Pickering emulsion from penetrating the skin. This is because the Pickering emulsion can include a reduced content of a surfactant required for emulsification, and thus inhibit the penetration of the aromatic compounds into the skin by the surfactant.

The aromatic compound is not particularly limited but is preferably an ultraviolet absorbing agent or a preservative.

The ultraviolet absorbing agent is not particularly limited, and examples thereof include ethylhexyl methoxycinnamate, octocrylene, dimethicodiethylbenzalmalonate, polysilicone-15, t-butylmethoxydibenzoylmethane, ethylhexyltriazone, hexyl diethylaminohydroxybenzoylbenzoate, bisethylhexyloxyphenol methoxyphenyl triazine, oxybenzone-3, methylene bisbenzotriazolyl tetramethylbutylphenol, phenylbenzimidazole sulfonic acid, homosalate, ethylhexyl salicylate, and other ultraviolet absorbing agents. As the ultraviolet absorbing agent used in the present invention, a single type or a combination of two or more types can be added. The ultraviolet absorbing agent is not particularly limited, but is preferably ethylhexyl methoxycinnamate, octocrylene, or hexyl diethylaminohydroxybenzoylbenzoate from the viewpoint of the stability of the emulsion.

The preservative is not particularly limited, and examples thereof include phenoxyethanol, benzoic acid and salts thereof, salicylic acid and salts thereof, phenol and salts thereof, dehydroacetic acid and salts thereof, paraoxybenzoic acid esters (for example, methylparaben, ethylparaben, butylparaben, and the like), chlorocresol, hexachlorophene, resorcin, isopropylmethylphenol, orthophenylphenol, benzalkonium chloride, chlorhexidine hydrochloride, chlorhexidine gluconate, alkyl isoquinolium bromide, trichlorocarbanilide, halocarban, photosensitive element No. 201, trichlorohydroxydiphenyl ether (triclosan), a methylchloroisothiazolinone/methylisothiazolinone solution, bisabolol, alkyl diaminoethyl glycine hydrochloride, trichloro salicylanilide (TCSA), tribromo salicylanilide (TBS), and other preservatives. As the preservative used in the present invention, a single type or a combination of two or more types can be added.

[Other Properties of Particle]

The average zeta potential of the particles of the present embodiment is calculated by electrophoresis of a composition mainly containing the particles, measuring a particle velocity with the laser Doppler velocimetry (for example, Zetasizer Nano ZSP manufactured by Malvern Panalytical Ltd.), and applying the following equation of Henry's law to the particle velocity.

[Math. 1]

$$U = \frac{2 \times \varepsilon \times z \times f(Ka)}{3 \times \eta}$$

Here, U represents electrophoretic mobility, ε represents relative permittivity of a particle dispersion medium, z represents zeta potential, f (Ka) represents a Henry's coefficient, and η represents the viscosity of the particle dispersion medium.

In more detail, see a method for measuring the average zeta potential described in Examples. The average zeta potential of the particles of the present embodiment can have an upper limit of +60 mV or +40 mV and a lower limit of +10 mV or +30 mV, and is preferably +10 mV or more and +60 mV or less, and more preferably +30 mV or more and +40 mV or less. Although the present invention is not bound by any theory, it is presumed that in a case where the average zeta potential of the particles of the present embodiment is within the above-described range, aggregation of the particles is inhibited by the particles repelling each other, thereby enhancing the storage stability of the particle. Furthermore, it is presumed that in a case where the average zeta potential of the particles of the present embodiment is within the above-described range, the particles are likely to adsorb to the interface between the hydrophilic medium and the hydrophobic medium in the emulsion composition, so that the emulsifying ability of the particles is improved, and the storage stability of the emulsion composition is enhanced.

In addition, in a dispersion containing the particles, the intensity of scattered light from the particles undergoing Brownian motion in a dispersion medium is measured to calculate the cumulant average diameter of the particles of the present embodiment from the temporal variation of the intensity obtained by the measurement (dynamic light scattering method). The cumulant average diameter of the particles of the present embodiment can have an upper limit of 500 nm or 450 nm and a lower limit of 100 nm or 150 nm, and is preferably 100 nm or more and 500 nm or less, and more preferably 150 nm or more and 450 nm or less, from the viewpoint of enhancing the storage stability of the particle, improving the emulsifying ability by the particle, and enhancing the storage stability of the emulsion composition.

Furthermore, a difference ($T_{0.01}$ - $T_1$) between an interfacial tension value ($T_{0.01}$) and an interfacial tension value ($T_1$), in which the interfacial tension value ($T_{0.01}$) is between an aqueous dispersion in which a content of the particles of the present embodiment is 0.01% by mass and decamethylcyclopentasiloxane, and the interfacial tension value ($T_1$) is between an aqueous dispersion in which a content of the particles of the present embodiment is 1% by mass and decamethylcyclopentasiloxane, is preferably 10 mN/m or more, and more preferably 15 mN/m or more, from the viewpoint of enhancing the emulsifying ability of the particles and ensuring the sufficient storage stability of the emulsion composition. In addition, the interfacial tension value ($T_1$) between the aqueous dispersion in which the content of the particles of the present embodiment is 1% by mass and decamethylcyclopentasiloxane is preferably 25 mN/m or less, more preferably 20 mN/m or less, and still more preferably 10 mN/m or more and 18 mN/m or less, from the viewpoint of enhancing the emulsifying ability of the particles and improving the storage stability of the emulsion composition.

In the present embodiment, the interfacial tension can be measured using a pendant drop method (hanging drop method). For the measurement, a contact angle meter (trade name: DMo-502, manufactured by Kyowa Interface Science Co., Ltd.) can be used, decamethylcyclopentasiloxane (cyclopentasiloxane, commonly called "D5", a specific gravity of 0.96 g/cm$^3$) can be used as a surrounding phase, and purified water, ion-exchanged water, pure water, and other solutions can be used as water used for the aqueous dispersion. In this case, measurement conditions of room temperature (25°C or similar temperature) and a humidity of 45%±10% can be employed.

Furthermore, in the present embodiment, the residual ratio of the insoluble fraction of the particles of the present embodiment after extraction with the Soxhlet extractor using chloroform as an extraction solvent is preferably 5% or more, and more preferably 6% or more and 15% or less. By adjusting the residual ratio of the insoluble fraction within the above-described range, the presence ratio of the hydrophobic segment and the hydrophilic segment of the particle of the present embodiment is suitably prepared, and the emulsifying ability by the particle of the present embodiment is improved, and the storage stability of the composition containing the particle is enhanced.

[Form and Properties of Composition Containing Particle]

The composition containing the particle of the present embodiment may be a dispersion substantially uniformly dispersed in a dispersion medium or a powder composition, and the form and properties thereof are not particularly limited. However, from the viewpoint of reducing the complexity of various process operations during production, the composition containing the particle of the present embodiment is preferably a dispersion substantially uniformly dispersed in a dispersion medium. As the dispersion medium, a reaction solvent obtained after the particle is synthesized may be left as it is and used as a dispersion medium, or the reaction solvent may be distilled off as necessary, another solvent may be

added thereto, and the resulting solvent may be used as a dispersion medium. From the viewpoint of reducing the complexity of various process operations during production, it is preferable to use the reaction solvent obtained after the particle is synthesized as a dispersion medium as it is. In a case where the reaction solvent after the particle synthesis is used as it is as a dispersion medium, it is preferable to remove unreacted monomers from the viewpoint of enhancing the storage stability and reducing the irritation. As for removing the unreacted monomers, although a specific method for implementing the removal is not particularly limited, it is preferable to additionally add a polymerization initiator to the composition containing the unreacted monomers from the viewpoint of reducing the complexity of various operations during production, enhancing the storage stability, and reducing the irritation. In that case, the amount of the polymerization initiator additionally added is preferably 0.1 times to 1 time the amount added during the particle synthesis. Although the present invention is not bound by any theory, it is presumed that the polymerization initiator is additionally added to convert the unreacted monomers into a dimer or higher polymer by a polymerization reaction, and the added polymerization initiator is, on the other hand, decomposed in the reaction solvent to form a stable decomposition product even when remaining. Incidentally, the mass of solid content in the dispersion containing the particle of the present embodiment is preferably 1% by mass or more, and more preferably 10% by mass or more and 50% by mass or less with respect to the total of the dispersion from the viewpoint of reducing costs in the production of the particle.

The dispersion medium for dispersing the particle of the present embodiment is not particularly limited, but from the viewpoint of enhancing the storage stability and reducing the irritation, it is preferable to use an aqueous medium, and it is more preferable to use water and an alcoholic medium. Specific examples of the alcoholic medium include lower alcohols, such as methanol, ethanol, and isopropyl alcohol, and low molecular weight diols and triols having 6 or less carbon atoms, such as 1,3-butylene glycol, glycerin, ethylene glycol, and propylene glycol. Among these, from the viewpoint of enhancing the storage stability, one or more selected from the group consisting of water and lower alcohols are preferable, and a medium containing at least water is preferable.

(Other Additives)

The composition containing the particle of the present embodiment may appropriately contain, as an optional component, various additives commonly used for the use as long as the object of the present invention is not impaired, in addition to various active ingredients (for example, cosmetic active ingredients; whitening agents, cell activators, anti-inflammatory agents, blood circulation promoters, skin astringents, antiseborrheic agents, and other active ingredients) which may be added as necessary according to the use of the composition. Examples of the additive include an antioxidant, a rust inhibitor, a gelling agent, a dispersant, a thickener, an oil agent, alcohol, ether, water, a pH adjusting agent, a stabilizer, a disinfectant, an antifungal agent, a preservative, a coloring agent, a chelating agent, a moisturizing agent, a pearlescent agent, perfume, an ultraviolet absorbing agent, an ultraviolet scattering agent, and other additives.

<Method for Producing Particle>

A method for producing the particle of the present embodiment is not particularly limited, and examples thereof include polymerizing the hydrophobic monomer and the hydrophilic monomer separately or sequentially (that is, the hydrophilic monomer is polymerized separately from the hydrophobic monomer; or the hydrophilic monomer is polymerized after completion of polymerization of the hydrophobic monomer), performing a particle formation, and other ways. The method for producing the particle of the present embodiment is not particularly limited, and may include, for example, the following particle formation step, and may include a modification step and other steps.

[(1) Particle Formation Step]

The particle formation step is not particularly limited, and examples thereof can include a step of forming a particle while polymerizing monomer components such as a hydrophobic monomer, for example, an emulsion polymerization method, a suspension polymerization method, a dispersion polymerization method, and other methods. In this polymerization, a polymerization reaction is preferably carried out in the presence of an emulsifier. The particle formation step may also be a particle formation step of polymerizing a monomer component by a solution polymerization method or other methods to obtain a reaction product, and then forming the obtained reaction product into a particle by a phase inversion emulsification method, a suspension method, or other methods.

However, from the viewpoint of reducing the complexity in production, a step of forming a monomer component into a particle while polymerizing the monomer component is preferable, an emulsion polymerization method, a suspension polymerization method, or a dispersion polymerization method is more preferable, and an emulsion polymerization method is still more preferable. Furthermore, among the emulsion polymerization methods, from the viewpoint that impurities such as an emulsifier are not mixed in the surface of the hydrophobic segment to be the core part, and the following modification step that may be followed is hardly affected, it is preferable to employ a soap-free emulsion

polymerization method, and a specific implementation technique of this soap-free emulsion polymerization method is not particularly limited, and can be implemented by a known method. For example, emulsion polymerization of the monomer component can be carried out in the presence of the polymerization initiator without using the emulsifier such as a surfactant, a polymeric emulsifier, or a reactive surfactant.

The solvent used in the particle formation step is not particularly limited, and examples thereof include an aqueous medium. The aqueous medium used in the particle formation step may contain water or alcohol from the viewpoint of enhancing the storage stability of the particle. The reaction temperature during the particle formation step is preferably set to a temperature equal to or lower than the boiling point of the solvent.

[(2) Modification Step]

In the modification step, for example, a part or the entirety of a surface of the hydrophobic segment is directly or indirectly covered with hydrophilic monomers or a polymer of the hydrophilic monomers. Alternatively, the modification step can also be expressed as a step of bonding or associating the hydrophobic monomers polymerized and the hydrophilic monomers polymerized.

Examples of the aspect of covering the surface of the hydrophobic segment directly with the hydrophilic monomers or other substance can include an aspect of bonding the hydrophobic monomer unit present on the surface of the hydrophobic segment to the hydrophilic monomer unit by a covalent bond, and more specifically, there are an aspect of covalently bonding an atomic group derived from a polymer of the hydrophilic monomer to an atomic group derived from the hydrophobic monomer unit present on the surface of the core part, and an aspect of covalently bonding vinyl acetate serving as a hydrophilic monomer to an atomic group derived from the hydrophobic monomer unit present on the surface of the core part, polymerizing vinyl acetate thereon, followed by performing ester hydrolysis of a polyvinyl acetate moiety to achieve saponification and thus a formation of a hydrophilic segment. However, as the aspect of covering the surface of the hydrophobic segment directly with the hydrophilic monomers or other substance, it is preferable to employ, from the viewpoint of improving emulsifying ability, an aspect of covalently bonding the polymer containing the hydrophilic monomer unit to an atomic group derived from the hydrophobic monomer unit present on the surface of the core part, it is more preferable that the polymer containing the hydrophilic monomer unit is polyvinyl alcohol, and it is still more preferable that the covalent bond is a carbon-carbon covalent bond or other bonded interaction formed by a radical reaction with a radical polymerization initiator.

In an aspect of covering the surface of the hydrophobic segment directly with the hydrophilic monomers or other substance, the thickness of a layer containing a polymer containing the hydrophilic monomer unit can be calculated. Specifically, an average particle diameter of the aspect of covalently bonding the polymer containing the hydrophilic monomer unit is subtracted by an average particle diameter of an aspect without covalently bonding the polymer containing the hydrophilic monomer unit, and the calculated result is further multiplied by 0.5 to calculate the thickness. In this calculation, the average particle diameter is determined as follows.

First, into a 50 mL Nunc tube, 20 mL of a polymer containing hydrophilic monomer units is dispensed and centrifuged using a centrifuge under a condition of 15,300 $\times$ g for 15 minutes. After centrifugation, the supernatant is removed, 20 mL of Elix water is added, the mixture is mixed and stirred with a vortex mixer until the precipitate is uniformly dispersed, and this operation is repeated 3 times, followed by washing. After the sample that has been washed three times is centrifuged again, Elix water is added, and the sample is redispersed so that the mass of solid content is 0.001% by mass; thereby, a sample for measuring the average particle diameter is prepared.

Next, the average particle diameter of the prepared sample is measured by using an appropriate molecular weight measuring device. For the measurement, analysis software may be appropriately used as necessary. The relative permittivity required for the measurement is a value of water at 25°C (78.5), the Henry's coefficient is 1.5, and the viscosity of a dispersion medium is a value of the viscosity of water at 25°C (0.89 mPa·s). The mass of solid content is calculated from a mass difference before and after drying, the mass difference being obtained by weighing each polymer containing hydrophilic monomer units and then performing heat drying or freeze drying to obtain the mass difference.

The thickness of the layer of a polymer containing the hydrophilic monomer unit calculated in this manner is not particularly limited, but the upper limit thereof can be 200 nm, 100 nm, 50 nm, 30 nm, or 20 nm, and the lower limit thereof can be 0.001 nm, 0.01 nm, 0.1 nm, 1 nm, or 5 nm from the viewpoint of enhancing the storage stability and ensuring the sufficient emulsifying ability by the particle. The thickness of the layer of a polymer containing an aqueous monomer unit is preferably 0.001 nm to 200 nm, more preferably 0.1 nm to 50 nm, and still more preferably 5 nm to 20 nm from the viewpoint of enhancing the storage stability and ensuring the sufficient emulsifying ability by the particle.

Examples of the aspect of covering the surface of the hydrophobic segment indirectly with the hydrophilic monomers or other substance include an aspect of causing an atomic group contained in the hydrophobic monomer unit present on the surface of the core part (hydrophobic segment) to interact with an atomic group contained in the hydrophilic monomer unit through an intermolecular force, electrostatic interaction, or other interactions. In this case, since the hydrophobic segment and the hydrophilic segment interact with each other as a result, a functional group or atomic group

may be optionally introduced into the hydrophobic segment and/or the hydrophilic segment in order to make the interaction between the hydrophobic segment and the hydrophilic segment easier. As a method for causing the atomic group contained in the hydrophobic monomer unit present on the surface of the hydrophobic segment to interact with the atomic group contained in the hydrophilic monomer unit through an intermolecular force, electrostatic interaction, or other interactions, it is preferable to employ a method for adding a polymer containing the hydrophilic monomer unit to the particle obtained in (1) the particle formation step, and introducing any functional group/atomic group as necessary while appropriately setting a temperature, pressure, concentration condition, and other factors of a mixed liquid, in which it is more preferable to perform heating, pressurizing, or concentrating the mixed liquid.

[Other Steps]

The method for producing the particle of the present embodiment may have other steps in addition to (1) the particle formation step and (2) the modification step. The "other steps" to be added are not particularly limited, and examples thereof include a washing step, a concentration step, and a drying step. For example, the washing step, the concentration step, and the drying step may be incorporated in at least one of the steps of (1) the particle formation step and (2) the modification step, or may be independent (temporally or spatially) from the steps of (1) the particle formation step and (2) the modification step.

The method for producing the particle of the present embodiment preferably has the washing step in order to reduce the irritation of the composition containing the obtained particle. The medium used in the washing step is not particularly limited, and an aqueous medium can be used. For example, in a case where the reaction solvent used in the particle formation step is an aqueous medium, a washing step can be carried out by repeating an operation of precipitating the particle of the present embodiment by centrifugation, removing a supernatant thereof, then adding the aqueous medium, and performing redispersion, or other operations. In addition, in a case where the reaction solvent used in the particle formation step is an organic solvent, a washing step may also be carried out by mixing an aqueous medium to which a salt such as brine is added with the reaction solvent, stirring the mixture, and separating the aqueous phase and the organic phase using a separatory funnel or other similar devices. Since the method for producing the particle of the present embodiment includes the washing step, the remaining amounts of the unreacted radical polymerization initiator, the decomposition products thereof, and the unreacted monomers in the medium can be reduced (for example, each concentration thereof can be less than 100 ppm).

The method for producing the particle of the present embodiment preferably includes a concentration step for increasing the concentration of the particle in the composition from the viewpoint of sufficiently exhibiting the emulsifying ability of the particle. Although not particularly limited, this concentration step may be carried out by subjecting the particle of the present embodiment to precipitation by centrifugation, removing a supernatant, and then adding a medium smaller than the mass and/or volume of the removed supernatant to perform redispersion, or may be carried out by subjecting the particle of the present embodiment to drying to obtain a powder, and then adding a medium smaller than the mass and/or volume of the removed medium by the drying to perform redispersion, or other ways.

In addition, the method for producing the particle of the present embodiment preferably includes the drying step in order to bring the state of the particle of the present embodiment into a powder state. The drying step can be carried out by evaporating and drying the medium containing the particles of the present embodiment. The specific technique employed in the drying step is not particularly limited, and examples thereof include hot air drying, infrared drying, fluidized bed drying, spray drying, freeze drying, reduced pressure drying, vacuum drying, and other drying methods.

The method for producing the particle of the present embodiment may have only one step as the particle formation step, but may employ a multi-step production method including other steps in addition to the particle formation step.

In a case where the method for producing the particle of the present embodiment is a method including a single step as the particle formation step, it is preferable to directly or indirectly cover a part or the entirety of the surface of the core part with hydrophilic monomers or other substance while forming the surface of the core part of the particle of the present embodiment to be hydrophobic by an appropriate modification of the hydrophobic segment or other treatment during the particle formation step.

In a case where the method for producing the particle of the present embodiment is a method including multiple steps with the particle formation step, it is preferable to employ a two-step method including (1) the particle formation step and (2) the modification step from the viewpoint of enhancing the storage stability and ensuring the sufficient emulsifying ability by the particle. In addition, in a case where the method for producing the particle of the present embodiment is a method including the multiple steps with the particle formation step, the method may be a method including three or more steps with other steps as a single step or a plurality of steps, and each step may be performed a plurality of times in an overlapping manner regardless of the order of each step. In a case where the method for producing the particle of the present embodiment is a method including two or more steps with (1) the particle formation step and (2) the modification step, it is preferable to form a hydrophobic segment (core part) in (1) the particle formation step, and directly or indirectly

cover a part or the entirety of the surface of the hydrophobic segment (core part) with hydrophilic monomers or other substance in (2) the modification step.

A more specific method for producing the particle is disclosed in detail in the following Examples.

<Use of Composition Containing Particle>

The use of the composition containing the particle of the present embodiment is not particularly limited, and the composition containing the particle of the present embodiment can be used as cosmetics, pharmaceuticals, quasi-drugs, inks (including inks and pigment dispersions contained in stationery such as writing instruments), paints, and other industrial uses. The composition containing the particle of the present embodiment can be appropriately mixed with various main ingredients/active ingredients and various additives in combination depending on the uses.

In a case where the composition containing the particle of the present embodiment is, for example, a cosmetic product, a pharmaceutical product, or a quasi-drug, various commonly used additives can be usually mixed in the composition as long as the effect of the present invention is not impaired. Examples of such additives include an antioxidant, a gelling agent, a thickener, an oil agent, alcohol, ether, water, a pH adjusting agent, a stabilizer, a disinfectant, an antifungal agent, a preservative, a coloring agent, a chelating agent, a moisturizing agent, a pearlescent agent, perfume, an ultraviolet absorbing agent, an ultraviolet scattering agent, and other additives.

Note that as a precaution, regarding the case where the composition containing the particle of the present embodiment is used as a cosmetic product, examples of the cosmetic active ingredient include various skin-beautifying ingredients (for example, whitening agents, cell activators, anti-inflammatory agents, blood circulation promoters, skin astringents, antiseborrheic agents, and other ingredients).

EXAMPLES

Hereinafter, the present invention will be described in detail with reference to Examples. Note that the following examples are described for illustration, and the content of the present invention is not limited to the following Examples in any way.

<Production Examples 1 and 2; Synthesis of Polymethyl Methacrylate (PMMA) Dispersion>

Methyl methacrylate (MMA) and pure water substituted with argon (trade name: water purified by Elix Essential UV [manufactured by Merck KGaA]; hereinafter, may be simply referred to as "Elix water", the resistivity of Elix water is 5 MΩ·cm or more) were added to a 30 mL transparent glass vial (trade name: SV-30, manufactured by Nippon Electric Glass Co., Ltd.) according to the formulation illustrated in Table 1 to obtain a total content of 30 mL. As a cationic radical polymerization initiator, 1 mL of a solution of ADIP whose counter anion was a chloride ion (hereinafter, referred to as ADIP-Cl) in methanol (MeOH) (23 mg of ADIP-Cl, 2mM) was added, and the mixture was stirred in a water bath at 60°C for 14 hours using a strong stirring bar to obtain a PMMA dispersion.

[Table 1]

Table 1: Synthesis conditions for Production Examples 1 and 2

| | Sample No. | Monomer | Polymerization initiator | Solvent | |
|---|---|---|---|---|---|
| | | MMA | ADIP-Cl | MeOH | Residue |
| | | (g) | (m g) | (mL) | |
| Production Example 1 | YT 358 | 45 | 23 | 1 | Purewater |
| Production Example 2 | YT 357 | 0.75 | 23 | 1 | Purewater |

<Production Examples 3 to 6; Synthesis of PMMA-PVA Dispersion>

To a 300 mL separable flask with a baffle plate were added the PMMA dispersion obtained according to the procedure of Production Examples 1 and 2 to obtain a final mass of solid content of 4.8 g; furthermore, a predetermined mass of polyvinyl alcohol having a predetermined degree of polymerization and a predetermined degree of saponification described in Table 2 were added, and Elix water was added thereto to obtain a total mass of 199 g. Thereafter, the solution was heated to 60°C while being stirred at a stirring rate of 450 rpm. Then, 1 mL of a 38 mg/mL ADIP-Cl solution was added, and the mixture was stirred at a stirring rate of 450 rpm at 60°C for 3 hours to obtain a PMMA-PVA dispersion. In addition, for Production Examples 3 to 6 even after storage at 50°C for 3 months, when the appearance and properties were observed,

it was confirmed that the particles were precipitated, but not coarse aggregates, and were returned to a uniform dispersion state merely by light shaking. Therefore, it was suggested that the storage stability of the obtained PMMA-PVA dispersion was good.

[Table 2]

| Table 2:Synthesis conditions for PM M A -PVA | | | | | | | | |
|---|---|---|---|---|---|---|---|---|
| | | Hydrophobic segment | | Polymerization initiator | PVA | | | Solvent |
| | Sample No. | P roducton Example | PMMA | ADIP-Cl | Degree of polymerization | Degree of saponification | PVA | Residue |
| | | | (g) | (m g) | | (%) | (g) | |
| Production Example 3 | YT320 | 1 | 4.8 | 38 | 500 | 87-89 | 0.7 | Pure water |
| Production Example 4 | YT321 | 1 | 4.8 | 38 | 500 | 87-89 | 2.7 | Pure water |
| Production Example 5 | YT373 | 1 | 4.8 | 38 | 500 | 80-84 | 2.7 | Pure water |
| Production Example 6 | YT372 | 2 | 4.8 | 38 | 500 | 87-89 | 2.7 | Pure water |

<Production Example 7; Synthesis of Fluorescent-labeled PMMA Dispersion>

To a 30 mL transparent glass vial (trade name: SV-30, manufactured by Nippon Electric Glass Co., Ltd.) were added 4.5 g of methyl methacrylate (MMA, 1.5 M), 16 mg of 3-(2-benzothiazolyl)-7-(diethylamino)coumarin (commonly called "coumarin-6", manufactured by Sigma-Aldrich Co. LLC., 1.5 mM), and 24 mL of argon-substituted Elix water. A 900 μL ADIP-Cl solution (23 mg of ADIP-Cl, 2 mM) in methanol (MeOH) was added and stirred in a water bath at 60°C for 14 hours using a strong stirring bar to obtain a fluorescent-labeled PMMA dispersion.

[Table 3]

| Table 3:Synthesis conditions for fluorescent-labeled PM M A em ulsion | | | | | | |
|---|---|---|---|---|---|---|
| | | Monomer | Fluorescent dye | Polymerization initiator | Solvent | |
| | Sample No. | MMA | Coumarin-6 | ADIP-Cl | MeOH | Residue |
| | | (g) | (m g) | (m g) | (mL) | |
| Production Example 7 | YT 313 | 45 | 16 | 23 | 1 | Pure water |

<Production Example 8; Synthesis of Fluorescent-labeled PMMA-PVA Dispersion>

To a 300 mL separable flask with a baffle plate were subsequently added the sample obtained in Production Example 7 to obtain a solid content of 4.8 g; 667mg of PVA (trade name: JP-05, manufactured by JAPAN VAM & POVAL CO., LTD., degree of saponification: 88 mol%, average degree of polymerization: 500), and 164 mL of argon-substituted Elix water. To the mixture was added 38 mg of ADIP-Cl (0.5 mM), and the resulting mixture was stirred at a stirring rate of 450 rpm at 60°C for 3 hours to obtain a fluorescent-labeled PMMA-PVA dispersion. Thereafter, the fluorescent-labeled PMMA-PVA dispersion was further subjected to washing and concentration operations.

[Table 4]

| Table 4: Synthesis conditions for fluorescent-labeled PM M A -PVA emulsion | | | | | | | | |
|---|---|---|---|---|---|---|---|---|
| | | Polym er | | Polymerization initiator | PVA | | | Solvent |
| | Sample No. | Production Example | Fluorescent-labeled PMMA (g) | ADIP-Cl (m g) | Degree of polymerization | Degree of saponification (%) | PVA (g) | Residue |
| Production Example 8 | YT317 | 7 | 4.8 | 38 | 500 | 87-89 | 0.7 | Pure water |

<Production Example 9; Synthesis of PMMA-PVA Dispersion>

To a 300 mL separable flask with a baffle plate were added the PMMA dispersion obtained according to the procedure of Production Example 1 to obtain a final mass of solid content of 4.8 g; furthermore, a predetermined mass of 2.7 g of PVA (trade name: JMR-3H, manufactured by JAPAN VAM & POVAL CO., LTD., degree of saponification: 78 mol%, average degree of polymerization: 110) was added, and Elix water was added thereto to obtain a total mass of 199 g. Thereafter, the solution was heated to 60°C while being stirred at a stirring rate of 450 rpm. Then, 1 mL of a 38 mg/mL ADIP-Cl solution was added, and the mixture was stirred at a stirring rate of 450 rpm at 60°C for 3 hours to obtain a PMMA-PVA dispersion. The PVA used in Production Example 9 has a smaller degree of polymerization and a lower degree of saponification than those used in Production Examples 3 to 6. It was also confirmed for Production Example 9 similar to Production Examples 3 to 6 even after storage at 50°C for 3 months that the particles were not coarse aggregates and were returned to a uniform dispersion state merely by light shaking. Therefore, it was suggested that the storage stability of the obtained PMMA-PVA dispersion was good, and it was also found that the PVA having a smaller degree of polymerization and a lower degree of saponification can be used for the synthesis of the PMMA-PVA dispersion.

<Washing and Concentration of PMMA-PVA Dispersion>

Unreacted PVA may be present in the PMMA-PVA dispersion. Examples of the unreacted PVA include PVA that is not bonded to particles. Furthermore, from the viewpoint of further improving the emulsifying ability of the particles, the mass of solid content of the PMMA-PVA dispersion is preferably increased, the mass of solid content is more preferably 4% by mass or more, still more preferably 7% by mass or more, and particularly preferably 10% by mass or more.

[Washing and Concentration Operations]

Into a 50 mL Nunc tube, 20 mL of a PMMA-PVA dispersion was dispensed and centrifuged using a centrifuge (trade name: MX-305, manufactured by Tommy Seiko Co., Ltd., rotor: AR510-04) under a condition of 15,300 × g for 15 minutes. The supernatant was removed, 20 mL of Elix water was added, and the mixture was mixed and stirred with a vortex mixer until the precipitate is uniformly dispersed. The above operation was repeated 3 times, as washing. The sample after the washing three times was centrifuged again, Elix water was added, and the sample was redispersed so that the mass of solid content was 10% by mass to obtain a PMMA-PVA concentrated dispersion.

<Analysis Method>

The average particle diameter, the dispersion degree (PdI), and the average zeta potential were measured by using a particle size/zeta potential/molecular weight measuring device (trade name: Zetasizer Nano ZSP, manufactured by Malvern Panalytical Ltd). As analysis software, Zetasizer Software 7.12 manufactured by Malvern Ltd., was used, and a relative permittivity of water (78.5) at 25°C, a Henry's coefficient of 1.5, and the viscosity of the dispersion medium of a value of the viscosity of water (0.89 mPa·s) at 25°C, which are necessary for the measurement of the particle diameter, PdI, and zeta potential, were respectively employed. The mass of solid content was calculated from a mass difference before and after drying, the mass difference being obtained by weighing each dispersion and then performing heat drying or freeze drying. The PVA addition rate was calculated as follows. A 1 g PMMA-PVA dispersion was centrifuged under a condition of 20,400 × g for 15 minutes. The supernatant liquid was collected by pipetting, was then transferred to an empty Eppendorf tube whose mass was measured in advance, and was freeze-dried. The mass of PVA remaining in the supernatant was calculated from the mass difference between the Eppendorf tube before and after freeze drying, and the calculation result was subtracted from the mass of charged PVA during the synthesis to calculate the PVA addition amount. According to the following equations, the PVA addition rate was calculated by dividing the PVA addition amount by the mass of the charged PMMA, and the resulting percentage was converted to be expressed by % by mass.

[Math. 2]

$$\text{PVA addition amount} = \text{mass of charged PVA} - \text{mass of PVA remaining in supernatant}$$

$$\text{PVA addition rate} = (\text{PVA addition amount} \div \text{PMMA charging amount}) \times 100$$

[Table 5]

| Table 5:D etails of produced sam ples | | | | | | | |
|---|---|---|---|---|---|---|---|
| | | D LS | | | PVA | | |
| | SampleNo. | Particle diam eter (nm) | PdI(-) | Zeta potential (m V ) | Addition rate % | Degree of polymerization | Degree of saponification (%) |
| Production Example 1 | YT358 | 426 | 0.068 | 53 | - | - | - |
| Production Example 2 | YT357 | 237 | 0.15 | 28 | - | - | - |
| Production Example 3 | YT320 | 453 | 0.12 | 31 | 3.7 | 500 | 87-89 |
| Production Example 4 | YT321 | 456 | 0.13 | 27 | 5.3 | 500 | 87-89 |
| Production Example 5 | YT373 | 424 | 0.03 | 28 | 9.3 | 500 | 80-84 |
| Production Example 6 | YT372 | 297 | 0.13 | 20 | 10.3 | 500 | 87-89 |
| Production Example 7 | YT 313 | 424 | 0.15 | 39 | - | - | - |
| Production Example 8 | YT317 | 438 | 0.18 | 22 | 4.6 | 500 | 87-89 |
| Production Example 9 | OG1428 | 447 | 0.32 | 48 | 3.8 | 110 | 78 |

<Experimental Example 1; Preparation of Emulsion Composition>

[Case of Using Dispersion of Production Example]

To a transparent glass vial (trade name: SV-20, manufactured by Nippon Electric Glass Co., Ltd., content: 20 mL, 27 mm × 55 mm) was added a PMMA or PMMA-PVA dispersion to obtain a mass of solid content of 1% by mass, 1 g of 1,3-butanediol (BG) and 7 g of mineral oil (trade name: MORESCO-WHITE P-70, manufactured by MORESCO Corporation)) were mixed, and Elix water was added thereto to obtain a total mass of 10 g (the mass ratio is oil : BG : water = 7 : 2 : 1, 1% by mass of PMMA or PMMA-PVA). The mixture was stirred by using a magnetic stirrer (trade name: NK-SA03, manufactured by Nagasawa Manufacturing Co., Ltd.) with a strong stirring bar (φ4.5 × 12 mm) at 2,000 rpm for 15 minutes. From the observation of the appearance and properties 1 minute after the completion of stirring, no phase separation or other changes were observed, and emulsification was confirmed.

[Case of Polyvinyl Alcohol (PVA)]

An attempt was made to prepare an emulsion composition by the same method as in [Case of Using Dispersion of Production Example]. An aqueous solution of PVA (trade name: JP-05, manufactured by JAPAN VAM & POVAL CO., LTD., degree of saponification: 88%, average degree of polymerization: 500) was prepared by adding PVA to reach 1% by mass, and the mixture was stirred.

The results are illustrated in Tables 6 and 7. In all the cases where PVA alone or particles not subjected to the PVA addition reaction were used (Comparative Examples 1 to 4), emulsification was not confirmed. On the other hand, in all the cases where the particles subjected to the addition reaction with PVA were used (Examples 1 to 5), it was confirmed that the particles were emulsified. Therefore, it was suggested that it is preferable to use a dispersion obtained by subjecting PMMA to the PVA addition reaction for emulsification.

Furthermore, in Examples 1 to 6, even after 3 months from emulsification at room temperature of 25°C, it was confirmed that no phase separation or other changes were observed from the observation of the appearance and

properties, and the emulsification was maintained. Therefore, it was suggested that the storage stability of the emulsion composition was also good.

[Table 6]

| Table 6: Result of emulsification test of Examples | | | | | | |
|---|---|---|---|---|---|---|
| Composition | Example 1 | Example 2 | Example 3 | Example 4 | Example 5 | Example 6 |
| Mineraloil | 7 | 7 | 7 | 7 | 7 | 7 |
| BG | 1 | 1 | 1 | 1 | 1 | 1 |
| Particle of Production Example 3 | 0.1 | - | - | - | - | - |
| Particle of Production Example 4 | - | 0.1 | - | - | - | - |
| Particle of Production Example 5 | - | - | 0.1 | - | - | - |
| Particle of Production Example 6 | - | - | - | 0.1 | - | - |
| Particle of Production Example 8 | - | - | - | - | 0.1 | - |
| Particle of Production Example 9 | - | - | - | - | - | 0.1 |
| Elix water | Residue | Residue | Residue | Residue | Residue | Residue |
| Total | 10 | 10 | 10 | 10 | 10 | 10 |
| Formation of em ulsion | ○ | ○ | ○ | ○ | ○ | ○ |
| Em ulsification type | 0/W | 0/W | 0/W | 0/W | 0/W | 0/W |
| (Unit: g) | | | | | | |

[Table 7]

| Table 7: Result of emulsification test of Comparative Examples | | | | |
|---|---|---|---|---|
| Composition | Comparative Example 1 | Comparative Example 2 | Comparative Example 3 | Comparative Example 4 |
| Mineraloil | 7 | 7 | 7 | 7 |
| BG | 1 | 1 | 1 | 1 |
| PVA | 0.1 | - | - | - |
| Particle of Production Example 1 | - | 0.1 | - | - |
| Particle of Production Exam ple 2 | - | - | 0.1 | - |
| Particle of Production Exam ple 7 | - | - | - | 0.1 |
| Purewater | Residue | Residue | Residue | Residue |
| Total | 10 | 10 | 10 | 10 |
| Formation of emulsion | × | × | × | × |
| Em ulsification type | - | - | - | - |
| (Unit: g) | | | | |

<Experimental Example 2; Confirmation of Pickering Emulsion>

[Method for Observing Emulsion with Fluorescent-Labeled Particle]

About 10 μL of the emulsion composition prepared in Examples 1 to 4 was placed on a film-bottom dish (manufactured by ibidi GmbH), and diluted with about 500 μL of water. Pipetting was avoided so as not to destabilize the emulsion too much upon dilution. Fluorescence was observed by using a confocal laser microscope (trade name: FV1000, manufactured by Olympus Corporation) at an excitation wavelength of 473 nm and a fluorescence wavelength of 485 nm to 585 nm with a 100 times oil immersion objective lens. The microscopic images are illustrated in Fig. 2. As a result of the

observation, it was confirmed that particles emitting fluorescence were present at the interface between the oil phase and the aqueous phase, and it was suggested that a Pickering emulsion was formed.

<Example 3; Measurement of Interfacial Tension Value>

[Method for Measuring Interfacial Tension Value]

An interfacial tension measurement was carried out by using a pendant drop method (hanging drop method). As a surrounding phase, decamethylcyclopentasiloxane (cyclopentasiloxane, commonly called "D5", a specific gravity of 0.96 g/cm$^3$, trade name: KF-995, manufactured by Shin-Etsu Chemical Co., Ltd.) was employed. A contact angle meter (trade name: DMo-502, manufactured by Kyowa Interface Science Co., Ltd.) was used under the conditions of a room temperature of 25°C and a humidity of 44%±6% to enclose an aqueous dispersion in a syringe, suspended droplets were then discharged into a surrounding phase through a syringe needle (needle size), and the shape of the suspended droplets was analyzed by a Young-Laplace method (analysis software name: FAMAS7.2.0, manufactured by Kyowa Interface Science Co., Ltd.) to calculate an interfacial tension value (static interfacial tension value). Respective aqueous dispersions in Production Examples 1 and 4 with a mass of solid content of 1% by mass, 0.1% by mass, 0.01% by mass, 0.001% by mass, and 0.0001% by mass were subjected to the measurement, and respective aqueous dispersions in Production Example 5, 6, and 9 with a mass of solid content of 1% by mass were subjected to the measurement. The measurement operation was repeated three times.

[Result of Measuring Interfacial Tension Value]

The result of the measurement of the interfacial tension value is illustrated in Fig. 3 and Table 8. It was found that, in Production Example 1, the change in the interfacial tension value was 1 mN/m or less when the dispersion concentration was within a range of 0.01% by mass to 1%, whereas in Production Example 4, a decrease of 20 mN/m or more in the interfacial tension value was observed, and the dispersion of Production Example 4 was excellent in the action of decreasing the interfacial tension with D5 at a mass of solid content of 0.01% by mass or more.

Furthermore, the measurement results for the aqueous dispersions having a mass of solid content of 1% by mass in Production Examples 1, 4, 5, 6, and 9 are illustrated in Table 8. The interfacial tension value of the dispersion of Production Example 1 was 28 mN/m, whereas the interfacial tension values of Production Examples 4, 5, 6, and 9 were all 20 mN/m. That is, it was found that the interfacial tension values of Production Examples 4, 5, 6, and 9 were smaller than that of Production Example 1, and the action of decreasing the interfacial tension was excellent.

[Table 8]

| Table 8: Result of measurement of interfacial tension value | |
|---|---|
| Sam ple nam e | Interfacial tension (m N /m) |
| Production Example 1 | 28.8± 0.3 |
| Production Example 4 | 16.0± 0.2 |
| Production Example 5 | 12.4± 0.1 |
| Production Example 6 | 16.6± 0.4 |
| Production Example 9 | 11.8 ± 0.1 |

<Example 4: Extraction Test and Analysis of Insoluble Fraction After Extraction by FT-IR>

[Method of Extraction Test]

In Production Examples 1 and 4, after the washing and concentration operations described in <Washing and Concentration of PMMA-PVA Dispersion> were carried out, 1.23 g and 1.98 g of dry powders obtained by freezing at -80°C and then drying with a vacuum freeze dryer were charged into a Soxhlet extractor together with 100 mL of chloroform serving as an extraction solvent, extraction was performed over 16 hours, and the masses of the extraction cylinder before and after extraction were measured.

Here, the reason why chloroform was used as the extraction solvent is that PMMA is dissolved in chloroform, but PVA is not dissolved. Specifically, 10 mL is sufficient as an amount of chloroform required to dissolve 1 g of PMMA prepared in Production Example 1, but 10,000 mL or more of chloroform is required to dissolve 1 g of PVA.

[Result of Extraction Test]

The result of the extraction test is illustrated in Table 9. In Production Example 1, the mass of the charged dry powder was 1.23 g, the cylinder mass before extraction was 2.97 g, and the cylindrical mass after extraction was 2.97 g. On the other hand, in Production Example 4, the mass of the charged dry powder was 1.98 g, the cylinder mass before extraction was 2.90 g, and the cylinder mass after extraction was 3.08 g, and in Production Example 4, the mass of insoluble fraction after extraction (= (cylinder mass after extraction) - (cylinder mass before extraction)) was 0.18 g. Furthermore, the residual ratio (%) of the insoluble fraction after extraction (= (mass of insoluble fraction)/(mass of charged dry powder) ×100) was 0% in Production Example 1 and 9.1% in Production Example 4. That is, in Production Example 1, no change in the masses before and after extraction of the extraction cylinder was observed, but in Production Example 4, the mass of the extraction cylinder after extraction was larger than that before extraction.

[Table 9]

| Table 9: Result of extraction test | | |
|---|---|---|
| | Production Example 1 | Production Example 4 |
| Mass of charged dry powder [g] | 1.23 | 1.98 |
| Cylinder mass before extraction [g] | 2.97 | 2.9 |
| Cylinder mass after extraction [g] | 2.97 | 3.08 |

From the result above, it was suggested not only that there was a change in the configuration of the portion corresponding to the hydrophobic segment between Production Examples 1 and 4, but also that PVA was added to the portion corresponding to the hydrophobic segment in Production Example 4.

[Method of Analysis of Insoluble Fraction After Extraction by FT-IR]

For the dispersions obtained in Production Examples 1 and 4, the insoluble fractions after the extraction test were taken out, and the remaining extraction solvent was vacuum-dried. The insoluble fractions that had been dried after the extraction test, and the freeze-dried powders of PVA and PMMA were each measured by Fourier transform infrared spectroscopy (FT-IR) (trade name: Nicolet6700, manufactured by ThermoFisher Scientific Inc.).

[Result of Analysis of Insoluble Fraction After Extraction by FT-IR]

The analysis result of the insoluble fraction after the extraction test by Fourier transform infrared spectroscopy (FT-IR) is illustrated in Figs. 4 and 5 (trade name: Nicolet6700, manufactured by ThermoFisher Scientific Inc.). In Fig. 5, the spectrum of Fig. 4 is enlarged in a similar proportion, and the axis labels are translated into Japanese. The three spectra in Figs. 4 and 5 indicate the FT-IR spectra of the insoluble fractions after the extraction test in Production Example 4, PVA, and the freeze-dried powder of Production Example 1 (PMMA), respectively. The spectrum of the insoluble fraction after the extraction test in Production Example 4 and the spectrum of PVA were identical in that the spectra had a peak at 3,200 $cm^{-1}$ to 3,500 $cm^{-1}$ (considered to be derived from alcohol O-H stretching) and a peak at 1,700 $cm^{-1}$ (considered to be derived from carboxylic acid C=O stretching). Furthermore, also in the overall characteristics of the spectra, the spectrum of the insoluble fraction after the extraction test in Production Example 4 and the spectrum of PVA were similar. These results suggested that the insoluble fraction contained a structure derived from PVA.

Furthermore, the insoluble fraction after the extraction test in Production Example 4, PVA, and the freeze-dried powder of Production Example 1 (PMMA) were each measured by Fourier transform infrared spectroscopy (FT-IR) (trade name: NicoletiS50, manufactured by ThermoFisher Scientific Inc).

[Result 2 of Analysis of Insoluble Fraction After Extraction by FT-IR]

The analysis result of the insoluble fraction after the extraction test by Fourier transform infrared spectroscopy (FT-IR) is illustrated in Fig. 6 (trade name: NicoletiS50, manufactured by ThermoFisher Scientific Inc). The four spectra in Fig. 6 are numbered from A to D in order from the top. The spectrum of A indicates an insoluble fraction after the extraction test in Production Example 4, the spectrum of B indicates PVA, the spectrum of C indicates a freeze-dried powder of Production Example 1 (PMMA), and the spectrum of D indicates a difference spectrum between A and B. The spectrum of A and the spectrum of B were identical in that the spectra had a peak at 3,200 $cm^{-1}$ to 3,500 $cm^{-1}$ (considered to be derived from alcohol O-H stretching) and a peak at 1,700 $cm^{-1}$ (considered to be derived from carboxylic acid C=O stretching).

Furthermore, also in the overall characteristics of the spectra, the spectra of A and B were similar, and when the spectrum of A was subjected to FT-IR spectral library searching (program name: OMNIC9.13, manufactured by ThermoFisher Scientific Inc.), PVA with a degree of saponification of 88 mol% was identified with a maximum hit rate of 76%. The overall characteristics of the spectrum of C and the spectrum of D are also very similar, and when the spectrum of D was subjected to FT-IR spectral library searching, PMMA was identified with a maximum hit rate of 85%. These results suggested that the insoluble fraction contained structures derived from PVA and PMMA.

<Comparative Example 1: Production of PMMA-PVA Dispersion Using Anionic Radical Polymerization Initiator>

[Production Examples 10 and 11: Production of PMMA-PVA Dispersion Using Anionic Radical Polymerization Initiator]

First, 3 g of methyl methacrylate (MMA) and argon-substituted Elix water were added to a 30 mL transparent glass vial (trade name: SV-30, manufactured by Nippon Electric Glass Co., Ltd.) according to the formulation illustrated in Table 1 described above to obtain a total content of 30 mL. To the mixture were added 41 mg of potassium peroxodisulfate (anionic radical polymerization initiator) and 1.7 μL of N,N,N',N'-tetramethylethylenediamine, and the resulting mixture was stirred in a water bath at 80°C for 4 hours with a strong stirring bar to obtain a PMMA dispersion polymerized using an anionic radical polymerization initiator.

To a 300 mL separable flask with a baffle plate was then added the PMMA dispersion polymerized using an anionic radical polymerization initiator to obtain a final mass of solid content of 4.8 g; 2.7 g of a predetermined polyvinyl alcohol described in Table 10 was added, and Elix water was added thereto to obtain a total mass of 199 g. Thereafter, the solution was heated to 60°C while being stirred at a stirring rate of 450 rpm. Furthermore, 27 mg of potassium peroxodisulfate and 1 μL of N,N,N',N'-tetramethylethylenediamine were added, and the mixture was stirred at a stirring rate of 450 rpm for 3 hours under the temperature condition described in Table 10. Finally, washing and concentration operations were carried out in the same manner as in the above-described [Washing and Concentration Operations] to obtain a PMMA-PVA dispersion polymerized using an anionic radical polymerization initiator.

[Table 10]

Table 10:D etails of produced particle and interfacial tension value

| | DLS | | | PVA | | | PVA addition reaction | | Interfacial tension |
|---|---|---|---|---|---|---|---|---|---|
| | Particle diameter (nm) | PdI(-) | Zeta potential (m V ) | Addition rate % | Degree of polymerization | Degree of saponification (%) | Reaction time (hour) | Reaction temperature (°C) | Interfacial tension (m N /m) |
| Production Example 10 | 386 | 0.07 | -32 | 2 .9 | 500 | 87-89 | 3 | 60 | 25.7± 0.1 |
| Production Example 11 | 394 | 0.15 | -39 | 0.3 | 500 | 87-89 | 3 | 80 | 25.7± 0.2 |

Comparative Examples 2: DLS Measurement and Interfacial Tension Value Measurement of PMMA-PVA Dispersion Using Anionic Radical Polymerization Initiator>

For Production Examples 10 and 11, the result of carrying out the DLS measurement and the interfacial measurement between the D5 and the dispersion with a concentration of 1% mass is illustrated in Table 10. In both Production Examples 10 and 11, the dispersion had a negative zeta potential and was an anionic dispersion. In addition, the interfacial tension value was 25.7 mN/m, which suggested that a decrease in the interfacial tension is less likely to occur as compared with Production Examples 4, 5, 6, and 9.

<Comparative Example 3: Preparation of Emulsion Composition of PMMA-PVA Dispersion Using Anionic Radical Polymerization Initiator>

In the method described in <Experimental Example 1; Preparation of Emulsion Composition>, the PMMA dispersion or the PMMA-PVA dispersion was replaced with the dispersion prepared in Production Example 10 or 11 to perform the preparation operation of the emulsion composition, and the appearance and properties 1 minute after the completion of stirring were observed. As a result, it was confirmed that phase separation or other changes were observed, and a uniformly emulsified emulsion composition was not obtained. Therefore, it was suggested that the particle of the present invention is necessary to have at least a cationic group.

<Production Examples 12 and 13; Synthesis of PMMA-PVA Particle Dispersion>

To a 300 mL separable flask with a baffle plate were added 42 g of MMA and 166 g of Elix water. The mixture was heated to 80°C while being stirred at 450 rpm using a sealing mixer UZU, and the entire amount of 0.22 g of 2,2'-azobis(2-methylpropionamidine)dihydrochloride (V-50, manufactured by FUJIFILM Wako Pure Chemical Corporation) dissolved in 2 g of pure water was charged thereto, and synthesized for 6 hours (Production Example 12). The mass of solid content of the obtained emulsion was 19.5% by mass.

Production Example 12 is different from Production Examples 1 and 2 in the synthesis container, stirring, time, and temperature condition. In addition, methanol is not used as a solvent. Furthermore, the condition is that the mass ratio of the MMA monomer to the total mass is high, and is different in that V-50 is used as a cationic polymerization initiator.

To a 300 mL separable flask with a baffle plate was then added the PMMA dispersion prepared in Production Example 12 to obtain a final mass of solid content of 30 g; 15 g of PVA (trade name: EG-05C, manufactured by Mitsubishi Chemical Group Corporation) and 0.07g of sodium acetate (manufactured by FUJIFILM Wako Pure Chemical Corporation) were added, and Elix water was added thereto to obtain a total mass of 198 g. Thereafter, the solution was heated to 80°C while being stirred at a stirring rate of 450 rpm, and stirring was continued for 1 hour to dissolve polyvinyl alcohol. After 1 hour from the heating, the entire amount of 0.19 g of ADIP-Cl dissolved in 2 g of pure water was charged into the solution whose temperature was adjusted to 60°C, and synthesized for 3 hours (Production Example 13).

In Production Example 13, the mass ratio of the PMMA dispersion and PVA in the total mass is higher than those in Production Examples 3 and 4. It is also different in that sodium acetate is added.

The result is illustrated in Table 11. Production Examples 12 and 13 were monodisperse cationic particles. In addition, the interfacial tension value of the sample after the washing operation of Production Example 13 was smaller than that of Production Example 1, and the action of decreasing the interfacial tension was excellent. The storage stability of Production Examples 12 and 13 was good.

[Table 11]

| Table 11:Details of produced sample | | | | | | | | |
|---|---|---|---|---|---|---|---|---|
| | | DLS | | | PVA | | | |
| | Sample No. | Particle diameter (nm) | PdI(-) | Zeta potential (mV) | Addition rate % | Degree of polymerization | Degree of saponification (%) | Interfacial tension (mN /m) |
| Production Example 12 | KR035 | 370 | 0.06 | 50 | - | - | - | - |
| Production Example 13 | KR037 | 437 | 0.07 | 13 | 4.3 | 500 | 87-89 | 17.0 |

<Production Examples 14 to 19: Synthesis of Dispersion of Copolymer of MMA and styrene (MMA-St copolymer) or Copolymer Dispersion of MMA and Butyl Acrylate (MMA-BA copolymer)>

A reagent was added to a 30 mL transparent glass vial (trade name: SV-30, manufactured by Nippon Electric Glass Co., Ltd.) to obtain a total internal content of 30 g as illustrated in Table 12, and synthesized by using a strong stirring bar at 60°C for 16 hours. No noticeable coarse aggregates were observed in the resulting dispersion under all synthesis conditions.

[Table 12]

| Table 12: Synthesis conditions for Production Examples 14 to 19 | | | | | | |
|---|---|---|---|---|---|---|
| | | Monomer | | | Polymerization initiator | Solvent |
| | Sample No. | MMA (g) | St (g) | BA (g) | ADIP-Cl (mg) | Residue |
| Production Example 14 | 0G1430 | 1.6 | 1.7 | 0 | 23 | Pure water |
| Production Example 15 | 0G1431 | 0.6 | 2.6 | 0 | 23 | Pure water |
| Production Example 16 | KR145 | 2 | 0 | 1 | 23 | Pure water |
| Production Example 17 | KR146 | 1.5 | 0 | 1.5 | 23 | Pure water |
| Production Example 18 | KR152 | 2.1 | 0 | 0.9 | 23 | Pure water |
| Production Example 19 | KR153 | 2.2 | 0 | 0.8 | 23 | Pure water |

These conditions are different from those of Production Examples 1 and 2 in that MMA and styrene (St) or MMA and butyl acrylate (BA) are added as monomers. In addition, methanol is not used as a solvent.

<Production Examples 20 to 25: PVA Modification Step for MMA-St Copolymer or MMA-BA Copolymer>

A reagent was added into a 300 mL separable flask with a baffle plate as illustrated in Table 13 to obtain a total internal content of 200 g. The particles of the MMA-St copolymer (Production Example 14 or Production Example 15) or the MMA-BA copolymer (Production Examples 16 to 19) were added to be 4.8 g, and PVA was charged as a powder and stirred at 60°C for 1 hour for dissolution. Subsequently, an initiator was added, and the mixture was synthesized using a sealing mixer UZU at 450 rpm at 60°C for 3 hours. After synthesis, the solid content of the emulsion and the PVA addition rate were measured. Furthermore, the obtained emulsion was centrifuged in a large centrifuge at 8,000 rpm for 30 minutes. The supernatant was removed, pure water was added thereto, and the solid content was redispersed to perform washing. Washing was repeated twice. After completion of the washing, the mixture was centrifuged again at 8,000 rpm for 30 minutes, the supernatant was removed, and pure water was then added to obtain a final concentration of a mass of solid content of 10% by weight, and the mixture was redispersed.

[Table 13]

| Table 13:Production conditions for P(MMA -Stcopolymeror MMA-BA copolymer)-PVA | | | | | | | | |
|---|---|---|---|---|---|---|---|---|
| | | Hydrophobic segment | | Polymerization initiator | PVA | | | Solvent |
| | Sample No. | Production Example | Polymer (g) | ADIF-Cl (mg) | Degree of polymerization | Degree of saponification (%) | PVA (g) | Residue |
| Production Example 20 | YT380 | 14 | 4.8 | 38 | 500 | 87-89 | 2.7 | Pure water |
| Production Example 21 | YT381 | 15 | 4.8 | 38 | 500 | 87-89 | 2.7 | Pure water |
| Production Example 22 | KR157 | 16 | 4.8 | 38 | 500 | 87-89 | 2.7 | Pure water |
| Production Example 23 | KR158 | 17 | 4.8 | 38 | 500 | 87-89 | 2.7 | Pure water |

(continued)

| Table 13:Production conditions for P(MMA -Stcopolymeror MMA-BA copolymer)-PVA | | | | | | | | |
|---|---|---|---|---|---|---|---|---|
| | | Hydrophobic segment | | Polymerization initiator | PVA | | | Solvent |
| | Sample No. | Production Example | Polymer (g) | ADIF-Cl (mg) | Degree of polymerization | Degree of saponification (%) | PVA (g) | Residue |
| Production Example 24 | KR159 | 18 | 4.8 | 38 | 500 | 87-89 | 2.7 | Pure water |
| Production Example25 | KR160 | 19 | 4.8 | 38 | 500 | 87-89 | 2.7 | Pure water |

<Details of Produced Particle>

The average particle diameter, the dispersion degree (PdI), the average zeta potential, the concentration of the mass of solid content, and the PVA addition rate were measured by the same methods as those described in <Analysis Method> described above.

($^1$H-NMR Measurement and Calculation of Copolymerization Ratio of MMA)

The composition ratio of MMA copolymerization (MMA unit, mol%) of the particles in Production Examples 14 to 19 was calculated by a relative quantitative method by proton nuclear magnetic resonance ($^1$H-NMR). Measurement was carried out with AV-300N (manufactured by Bruker Corporation). After dry heating and drying, 2 mg of the sample was weighed and dissolved in 1 mL of deuterated chloroform (0.05% by weight of tetramethylsilane, manufactured by Tokyo Chemical Industry Co., Ltd.) for overnight, and the resulting solution was sealed in an NMR tube and measured (300 MHz, 16 integrations). The measurement standards were CHCl3 (7.26 ppm) and tetramethylsilane (0.00 ppm). Based on the obtained spectrum, the MMA unit for the entire copolymer composition was calculated from the signal intensities of structures derived from MMA, styrene, and butyl acrylate.

(Measurement of Glass Transition Temperature by Differential Scanning Calorimetry)

For Production Examples 14 to 19, the glass transition temperature of the freeze-dried powder after the washing operation was measured by differential scanning calorimetry (DSC) (measurement conditions/heating rate: 20°C/min, measurement temperature range: - 50°C to 100°C, nitrogen flow: 50mL/min), reference: empty pan (aluminum crimp), measurement device: EXSTAR DSC7020 (Hitachi High-Tech Science Corporation)). More specific measurement methods of the DSC method conformed to the method described in JIS K 7121 (1987).

The measurement result is illustrated in Table 14 below.

[Table 14]

| Table 14:Details of produced particle | | | | | | | | | | |
|---|---|---|---|---|---|---|---|---|---|---|
| | | DLS | | | PVA | | | | | |
| | Sample No. | Particle diameter | PdI | Zeta potential | Addition rate | Degree of polymerization | Degree of saponification | MMA unit | Glass transition temperature | Interfacial tension |
| | | (nm) | (-) | (mV) | % | | (%) | (mol%) | °C | (mN/m) |
| Production Example 14 | 0G1430 | 318 | 0.070 | 50 | - | - | - | 48 | 98.9 | - |
| Production Example 15 | 0G1431 | 369 | 0.050 | 50 | - | - | - | 17 | 99.0 | - |
| Production Example 16 | KR145 | 397 | 0.13 | 46 | - | - | - | 52 | 45.6 | - |
| Production Example 17 | KR146 | 402 | 0.11 | 48 | - | - | - | 37 | 37.0 | - |
| Production Example 18 | KR152 | 397 | 0.13 | 49 | - | - | - | 51 | 48.1 | - |
| Production Example 19 | KR153 | 393 | 0.13 | 48 | - | - | - | 57 | 49.9 | - |
| Production Example 20 | YT380 | 350 | 0.002 | 32 | 2.8 | 500 | 87-89 | - | - | 16.7± 0.0 |
| Production Example 21 | YT381 | 421 | 0.040 | 17 | 3.6 | 500 | 87-89 | - | - | 16.9± 0.1 |
| Production Example 22 | KR157 | 429 | 0.12 | 38 | 6.6 | 500 | 87-89 | - | - | 16.8± 0.2 |
| Production Example 23 | KR158 | 445 | 0.026 | 46 | 4.3 | 500 | 87-89 | - | - | 16.6± 0.1 |
| Production Example 24 | KR159 | 454 | 0.084 | 48 | 4.8 | 500 | 87-89 | - | - | 17.0± 0.1 |
| Production Example 25 | KR160 | 430 | 0.051 | 43 | 7.7 | 500 | 87-89 | - | - | 17.2± 0.2 |

<Preparation of Emulsion Composition>

Production Example 4 and Production Examples 20 to 25 were each added to a 300 mL disposable cup to obtain a mass of solid content of 1% by mass, 10 g of BG and Elix water were weighed according to the mass described in Table 15, and the mixture was stirred at 2,000 rpm by a homogenizer (trade name: ultra-high speed emulsification and dispersion machine, small table-top machine LABOLUTION (R) for research, stirring section: MARKII 2.5 type, manufactured by PRIMIX Corporation). Subsequently, 140 g of decamethylcyclopentasiloxane (trade name: KF-995, manufactured by Shin-Etsu Chemical Co., Ltd.) was added gradually. After completion of the addition, the mixture was stirred at 2,000 rpm for 1 minute, and then stirred at 4,500 rpm for 3 minutes to prepare an emulsion. From the observation of the appearance and properties 1 minute after the completion of stirring, no phase separation or other changes were observed, and emulsification was confirmed.

The result is illustrated in Table 15. Emulsification was confirmed in all of Examples 7 to 13, and the emulsification type was O/W.

[Table 15]

Table 15 : Result of emulsification test in Examples

| Composition | Example 7 | Example 8 | Example 9 | Example 10 | Example 11 | Example 12 | Example 13 |
|---|---|---|---|---|---|---|---|
| Decamethylcyclopentasiloxane | 140 | 140 | 140 | 140 | 140 | 140 | 140 |
| BG | 10 | 10 | 10 | 10 | 10 | 10 | 10 |
| Particle of Production Example 4 | 2 | | | | | | |
| Particle of Production Example 20 | - | 2 | - | - | - | - | - |
| Particle of Production Example 21 | - | - | 2 | - | - | - | - |
| Particle of Production Example 22 | - | - | - | 2 | - | - | - |
| Particle of Production Example 23 | - | - | - | - | 2 | - | - |
| Particle of Production Example 24 | - | - | - | - | - | 2 | - |
| Particle of Production Example 25 | - | - | - | - | - | - | 2 |
| Elix water | Residue | Residue | Residue | Residue | Residue | Residue | Residue |
| Total | 200 | 200 | 200 | 200 | 200 | 200 | 200 |
| Formation of emulsion | ○ | ○ | ○ | ○ | ○ | ○ | ○ |
| Emulsification type | 0 /W | 0 /W | 0 /W | 0 /W | 0 /W | 0 /W | 0 /W |

(Unit:g)

<Measurement of Molecular Weight by Gel Permeation Chromatography>

A gel permeation chromatography (GPC) analysis was carried out on a dried product of PVA (trade name: JP-05, manufactured by JAPAN VAM & POVAL CO., LTD., degree of saponification: 88 mol%, average degree of polymerization: 500) and the insoluble fraction after extraction in Production Example 1, Production Example 4 and Production Example 4 (the insoluble fraction after extraction, which was obtained after Production Example 4 was subjected to the extraction test in Example 4).

PVA, the freeze-dried products of Production Example 1 and Production Example 4, and the dried product of the insoluble fraction after extraction in Production Example 4 were dissolved in hexafluoroisopropanol (10 mmol/L sodium trifluoroacetate solution) to obtain 0.2 w/v%. The pretreatment was performed with a 0.2 μm PTFE membrane filter. As the measuring apparatus, GPC-104 (manufactured by SHOKO Scientific Co., Ltd.) was used as an RI detector. As the column, two coupled Shodex GPC LF-404 columns (manufactured by Showa Denko K.K.) were used. The column temperature

was set to 40°C, the flow rate was set to 0.3 mL/min, and the injection amount was set to 10 μL. Polymethyl methacrylate (PMMA) was used as a standard substance, a calibration curve was prepared, and the molecular weight of each sample was determined.

The result is illustrated in Table 16. As a result of the GPC analysis of Production Examples 1 and 4, the number-average molecular weights were 150,000 and 130,000, respectively, and the weight-average molecular weights were 580,000 and 560,000, respectively. In addition, as a result of the GPC analysis of the insoluble fraction after extraction in Production Example 4 and PVA, the number-average molecular weights were 75,000 and 39,000, respectively, and the weight-average molecular weights were 150,000 and 82,000, respectively.

[Table 16]

| Table 16:Measurement of molecular weight by gel permeation chrom atography | | | | |
|---|---|---|---|---|
| | Sample No. | Number-average molecular weight | Weight-average molecular weight | Polydispersity |
| | | Mn ($\times 10^4$ g/mol) | Mw ($\times 10^4$ g/mol) | Mw/Mn |
| Production Example 1 | YT 358 | 15 | 58 | 3.9 |
| Production Example 4 | YT 321 | 13 | 56 | 4.3 |
| Insoluble fraction after extraction in Production Example 4 | - | 7.5 | 15 | 2.0 |
| PVA (JP-05) | - | 3.9 | 8.2 | 2.1 |

From this result, when PVA and the dried product (PMMA-PVA dispersion) of the insoluble fraction after extraction in Production Example 4 were compared, the number-average molecular weight and the weight-average molecular weight of the dried product of the insoluble fraction after extraction in Production Example 4 were larger. In the extraction test in Example 4, since PMMA is dissolved and PVA is not dissolved in chloroform used as a solvent, PMMA in the PMMA-PVA dispersion is extracted, and is not contained in the dried product of the insoluble fraction after extraction. On the other hand, as described above, since there was a difference in the number-average molecular weight and the weight-average molecular weight, it was found that in Production Example 4, the molecular weight increased due to bonding of PVA to PMMA or the like.

<Preparation of Formulated Composition Containing Ultraviolet Absorbing Agent>

(Reference Example 1)

2-Ethylhexyl 2-cyano-3,3-diphenylacrylate (hereinafter, referred to as octocrylene, manufactured by Tokyo Chemical Industry Co., Ltd.), which is an ultraviolet absorbing agent described in Table 17, and pentaerythritol tetra(2-ethylhexanoate) (manufactured by FUJIFILM Wako Pure Chemical Corporation) were mixed to obtain an oily composition.

(Reference Examples 2 to 4)

Components other than octocrylene that is an ultraviolet absorbing agent and pentaerythritol tetra(2-ethylhexanoate) (manufactured by FUJIFILM Wako Pure Chemical Corporation) described in Table 17 were added, and the mixture was stirred at 2,000 rpm by a homogenizer. Thereafter, the oily composition (Reference Example 1) was gradually added. After completion of the addition, the mixture was stirred at 2,000 rpm for 1 minute, and then stirred at 4,500 rpm for 3 minutes to obtain an emulsion composition. In Reference Examples 2 to 4, emulsification was confirmed as no phase separation or other changes were recognized by the observation of the appearance and properties 1 minute after the completion of stirring.

[Table 17]

| Table 17 : Formulation conditions including ultraviolet absorbing agent and result of emulsification test | | | | |
|---|---|---|---|---|
| | Reference Example 1 | Reference Example 2 | Reference Example 3 | Reference Example 4 |
| LotNo. | 230323-1 | 230323-2 | 230323-3 | 230323-4 |

(continued)

| Table 17 : Formulation conditions including ultraviolet absorbing agent and result of emulsification test | | | | |
|---|---|---|---|---|
| | Reference Example 1 | Reference Example 2 | Reference Example 3 | Reference Example 4 |
| Composition | | | | |
| Octocrylene | 10 | 10 | 10 | 10 |
| Pentaerythritoltetra(2-ethylhexanoate) | 130 | 130 | 130 | 130 |
| BG | 0 | 20 | 20 | 20 |
| PVA | 0 | 0 | 4 | 2 |
| Particle of Production Example 4 | 0 | 0 | 0 | 2 |
| Tween 80 | 0 | 4 | 0 | 0 |
| Elixwater | 0 | 36 | 36 | 36 |
| Total | 140 | 200 | 200 | 200 |
| Formation of emulsion | - | O | O | O |
| Emulsification type | - | 0 /W | 0 /W | 0 /W |
| | | | | (Unit:g) |

That is, in Reference Examples 2 to 4, the oil phase containing the organic compound such as the ultraviolet absorbing agent was emulsified in an O/W emulsification type.

<Measurement of Thickness of PVA Layer>

Production Examples 1, 4, and 5 were washed and concentrated by [Washing and Concentration Operations] described above, followed by the preparation with pure water to obtain a mass of solid content of 0.001% by mass. The thickness of the PVA layer was measured by using a particle size/zeta potential/molecular weight measuring device (trade name: Zetasizer Nano ZSP, manufactured by Malvern Panalytical Ltd), and Zetasizer Software 7.12 manufactured by Malvern Ltd. was used as analysis software. As the average particle diameter, an average value of three measurements was employed.

The thickness of the PVA layer was calculated by the following equation with reference to Non Patent Document (Colloids and Surfaces A: Physicochemical and Engineering Aspects, 2014 August, 456, 139-145) and Non Patent Document (Journal of Colloid and Interface Science, 2020 March, 562, 204-212).

[Math. 3]

Thickness of PVA layer = 0.5 × {(average particle diameter of PMMA-PVA dispersion) - (average particle diameter of PMMA dispersion)}

The result is illustrated in Table 18. In Production Examples 4 and 5, the thicknesses of the PVA layers were calculated to be 15 nm and 16 nm, respectively. The average particle diameter of the PMMA-PVA dispersion was larger than that of the PMMA dispersion, and since the formation of the PVA layer was recognized on the surface of the PMMA particle, it was suggested that PVA was added to the surface of the PMMA particle even after the washing operation.

[Table 18]

| **Table 18: Measurement result of thickness of PVA adsorption layer** | | | |
|---|---|---|---|
| Sample name | Sample No. | Average particle diameter (nm) | Thickness of PVA layer (nm) |
| Production Example 1 | YT358 | 410 | - |
| Production Example 4 | YT321 | 440 | 15 |

(continued)

| Table 18: Measurement result of thickness of PVA adsorption layer | | | |
|---|---|---|---|
| Sample name | Sample No. | Average particle diameter (nm) | Thickness of PVA layer (nm) |
| Production Example 5 | YT373 | 441 | 16 |

## Claims

1. A particle comprising:

   a hydrophobic segment that contains a hydrophobic monomer unit and has a cationic group; and
   a hydrophilic segment that contains at least a hydrophilic monomer unit represented by Formula (I) below,

   [Chem. 1]

   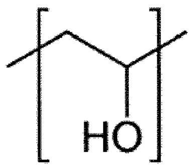

   (I)

2. The particle according to claim 1, wherein a part or entirety of a surface of the hydrophobic segment is directly or indirectly covered with the hydrophilic monomer or a polymer of the hydrophilic monomer.

3. The particle according to claim 1 or 2, wherein the cationic group is derived from a cationic radical polymerization initiator.

4. The particle according to claim 1 or 2, wherein the hydrophobic monomer unit is a monomer unit having an ethylenically unsaturated double bond.

5. The particle according to claim 4, wherein the monomer unit having an ethylenically unsaturated double bond is a (meth)acrylic acid monomer unit.

6. The particle according to claim 4, wherein the monomer unit having an ethylenically unsaturated double bond is a styrene monomer unit.

7. The particle according to claim 3, wherein the cationic radical polymerization initiator is at least one selected from the group consisting of 2,2'-[diazene-1,2-diylbis(propane-2,2-diyl)]bis(1,3-dimethyl-4,5-dihydro-1H-imidazole-3-ium) =ditrifluoromethanesulfonate (ADIP), 2,2'-[diazene-1,2-diylbis(propane-2,2-diyl)]bis(1,3-dimethyl-4,5-dihydro-1H-imidazole-3-ium)=dichloride (ADIP-Cl), 2,2'-azobis(2-methylpropionamidine)dihydrochloride (V-50), 2,2'-azobis [2-(2-imidazolin-2-yl)propane]dihydrochloride (VA-044), and 2,2'-azobis[2-(2-imidazoline-2-yl)propane] (VA-061).

8. The particle according to claim 1 or 2, wherein the hydrophilic segment further contains a monomer unit represented by Formula (II) below, and a ratioof a number of the monomer unit represented by Formula (I) to a total of the number of the monomer unit represented by Formula (I) and a number of the monomer unit represented by Formula (II) (degree of saponification) is 70% or more and 99% or less,

[Chem. 2]

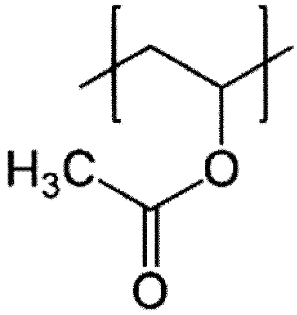

(I I)

9. The particle according to claim 8, wherein the ratio of the number of the monomer unit represented by Formula (I) to a total of the number of the monomer unit represented by Formula (I) and the number of the monomer unit represented by Formula (II) (degree of saponification) is 78% or more and 90% or less.

10. The particle according to claim 1 or 2, wherein a mass corresponding to the hydrophilic segment is equivalent to 1 part by mass or more and 6 parts by mass or less with respect to 100 parts by mass of a mass corresponding to the hydrophobic segment.

11. The particle according to claim 1 or 2, wherein the particle has an average zeta potential of +10 mV or more and +60 mV or less.

12. The particle according to claim 1 or 2, wherein the particle has a cumulant average diameter of 100 nm or more and 500 nm or less.

13. The particle according to claim 2, wherein the hydrophobic monomer unit and the hydrophilic monomer unit are bonded by a covalent bond, and the part or entirety of the surface of the hydrophobic segment is directly covered with the hydrophilic monomer or a polymer of the hydrophilic monomer.

14. The particle according to claim 2, wherein the hydrophobic monomer unit and the hydrophilic monomer unit are bonded by an intermolecular force, and the part or entirety of the surface of the hydrophobic segment is indirectly covered with the hydrophilic monomer or a polymer of the hydrophilic monomer.

15. The particle according to claim 14, wherein a bonding by the intermolecular force is induced by heat treatment.

16. The particle according to claim 1 or 2, wherein a difference ($T_{0.01}$ - $T_1$) between an interfacial tension value ($T_{0.01}$) and an interfacial tension value ($T_1$) is 10 mN/m or more, the interfacial tension value ($T_{0.01}$) being between an aqueous dispersion containing 0.01% by mass of the particle and decamethylcyclopentasiloxane, and the interfacial tension value ($T_1$) being between an aqueous dispersion containing 1% by mass of the particle and decamethylcyclopentasiloxane.

17. The particle according to claim 1 or 2, wherein the interfacial tension value ($T_{0.01}$) is 25 mN/m or less.

18. The particle according to claim 1 or 2, wherein a relative ratio between a molecular weight of the hydrophobic segment alone and a molecular weight of the hydrophilic segment alone per particle unit is such that the hydrophilic segment corresponds to 1 or more and 6 or less when the hydrophobic segment corresponds to 100.

19. The particle according to claim 1 or 2, wherein a residual ratio of an insoluble fraction after extraction with a Soxhlet extractor using chloroform as an extraction solvent is 5% or more.

20. The particle according to claim 1 or 2, wherein the particle is used to form a Pickering emulsion.

21. A method for producing the particle according to claim 1 or 2, the method comprising the steps of:

polymerizing a hydrophobic monomer in a presence of a cationic radical polymerization initiator and an emulsifier;
polymerizing a hydrophilic monomer separately from the hydrophobic monomer or after completion of polymerizing the hydrophobic monomer; and
bonding or associating the hydrophobic monomer polymerized and the hydrophilic monomer polymerized.

**22.** The particle according to claim 1 or 2, wherein the particle contains a polymer having a hydrophobic segment and a hydrophilic segment.

**23.** The particle according to claim 22, wherein the polymer having a hydrophobic segment and a hydrophilic segment has a number-average molecular weight of 1,000 or more and 500,000 or less.

**24.** The particle according to claim 22, wherein the polymer having a hydrophobic segment and a hydrophilic segment has a glass transition temperature of 30°C or higher and 200°C or lower.

**25.** The particle according to claim 1 or 2, wherein the hydrophobic monomer is at least one selected from the group consisting of methyl methacrylate, styrene, and butyl acrylate.

**26.** The particle according to claim 20, wherein the Pickering emulsion is for an application to a skin.

**27.** The particle according to claim 20, wherein the Pickering emulsion contains an aromatic compound.

**28.** The particle according to claim 27, wherein the aromatic compound is an ultraviolet absorbing agent or a preservative.

**29.** The particle according to claim 20, wherein the Pickering emulsion is a Pickering emulsion that prevents the aromatic compounds contained in the Pickering emulsion from penetrating a skin.

**30.** The particle according to claim 13, wherein the hydrophobic monomer unit and the hydrophilic monomer unit are bonded by a covalent bond, and the part or entirety of the surface of the hydrophobic segment is directly covered with a polymer of the hydrophilic monomer, and the polymer of the hydrophilic monomer constitutes a layer having a thickness of 0.001 nm to 200 nm.

FIG. 1

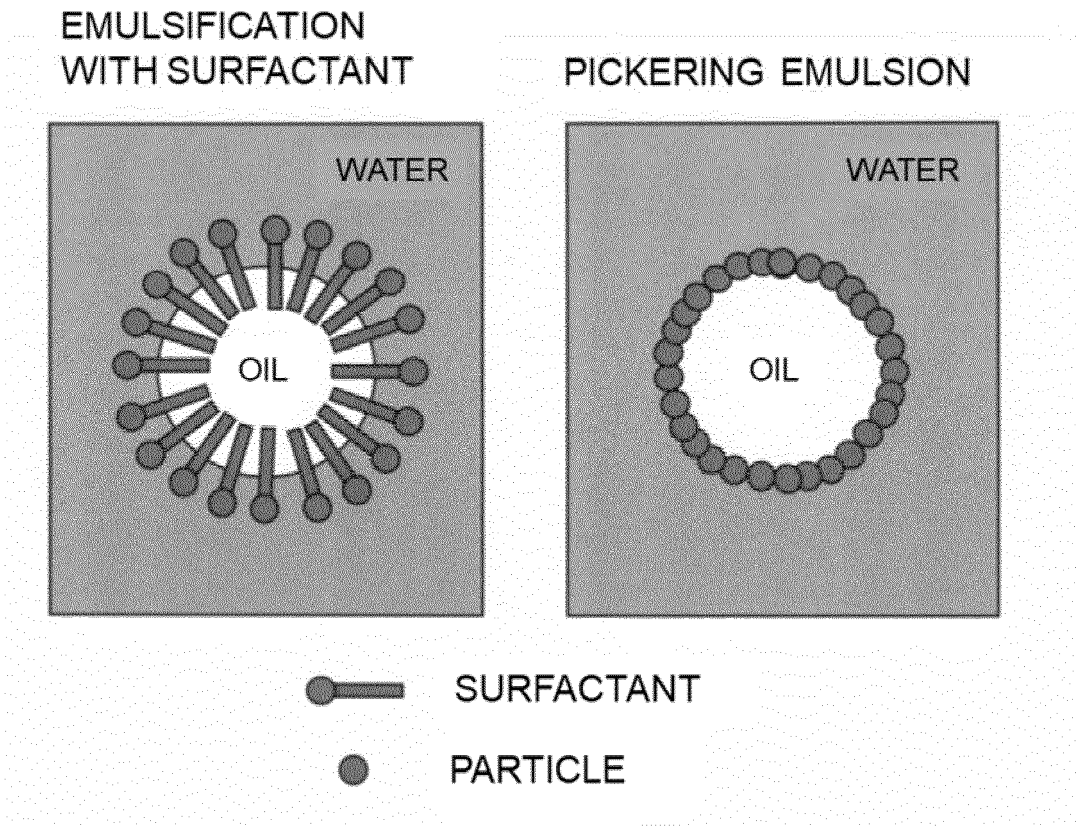
EMULSIFICATION
WITH SURFACTANT
PICKERING EMULSION
WATER
OIL
WATER
OIL
SURFACTANT
PARTICLE

FIG. 2

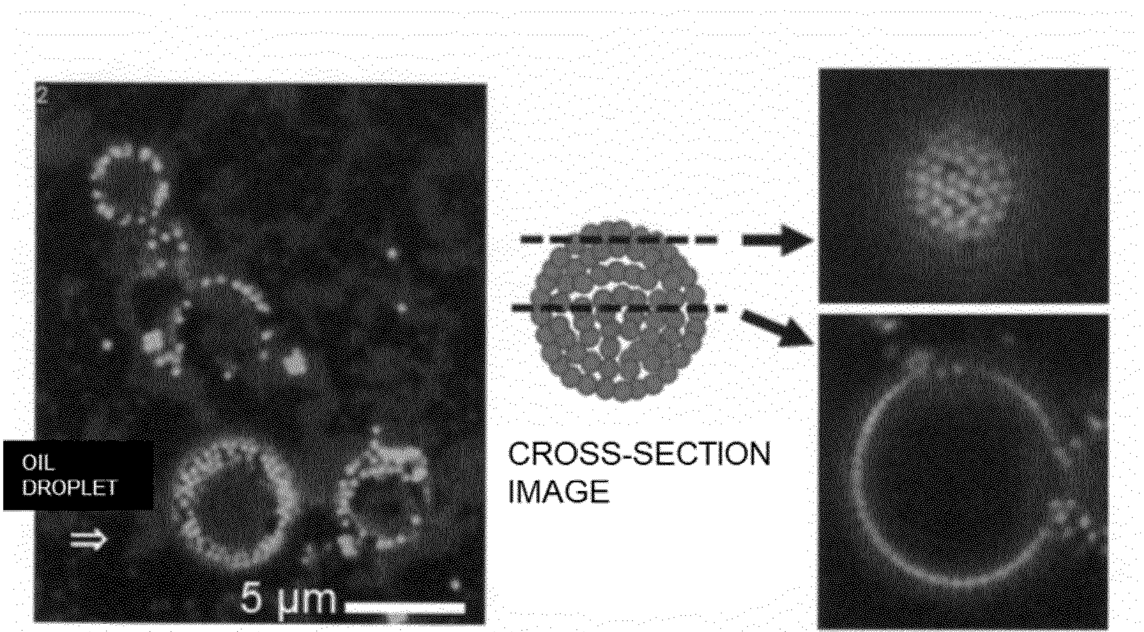

FIG. 3

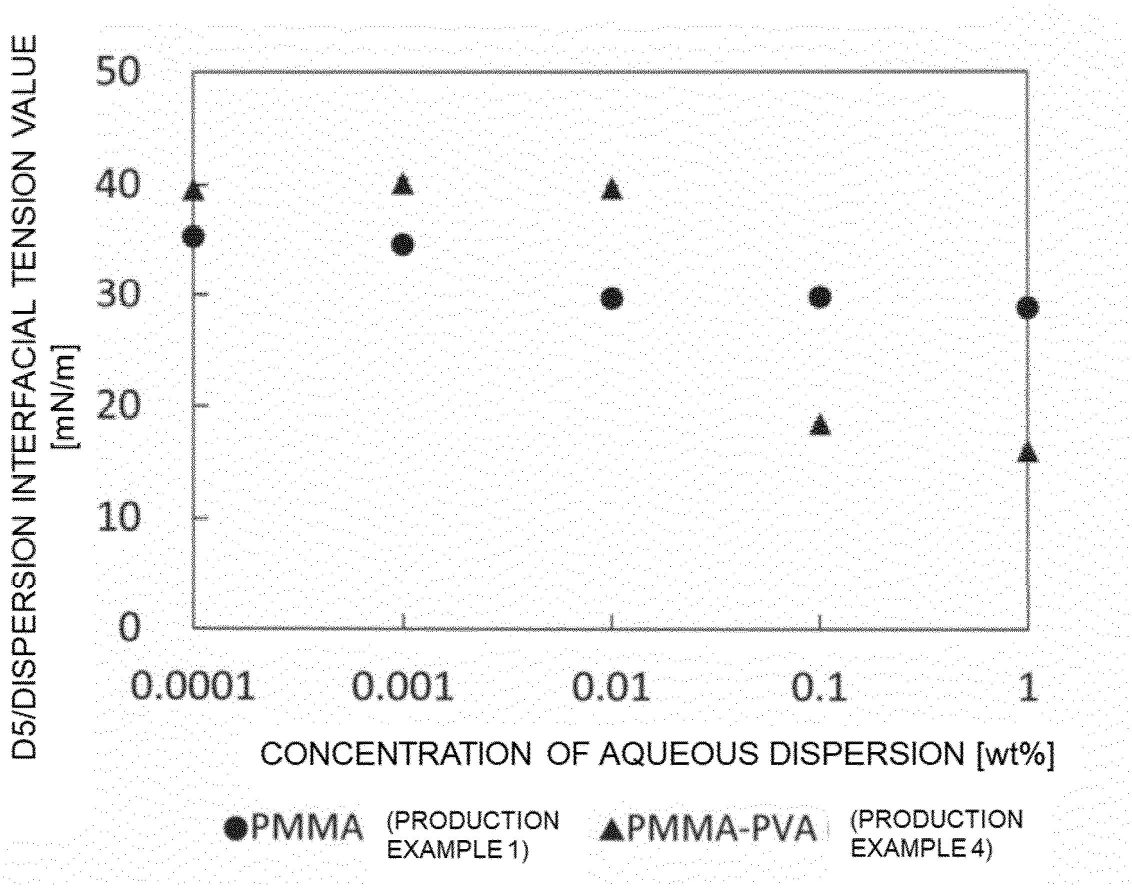
D5/DISPERSION INTERFACIAL TENSION VALUE [mN/m]
50
40
30
20
10
0
0.0001
0.001
0.01
0.1
1
CONCENTRATION OF AQUEOUS DISPERSION [wt%]
●PMMA (PRODUCTION EXAMPLE 1)
▲PMMA-PVA (PRODUCTION EXAMPLE 4)

FIG. 4

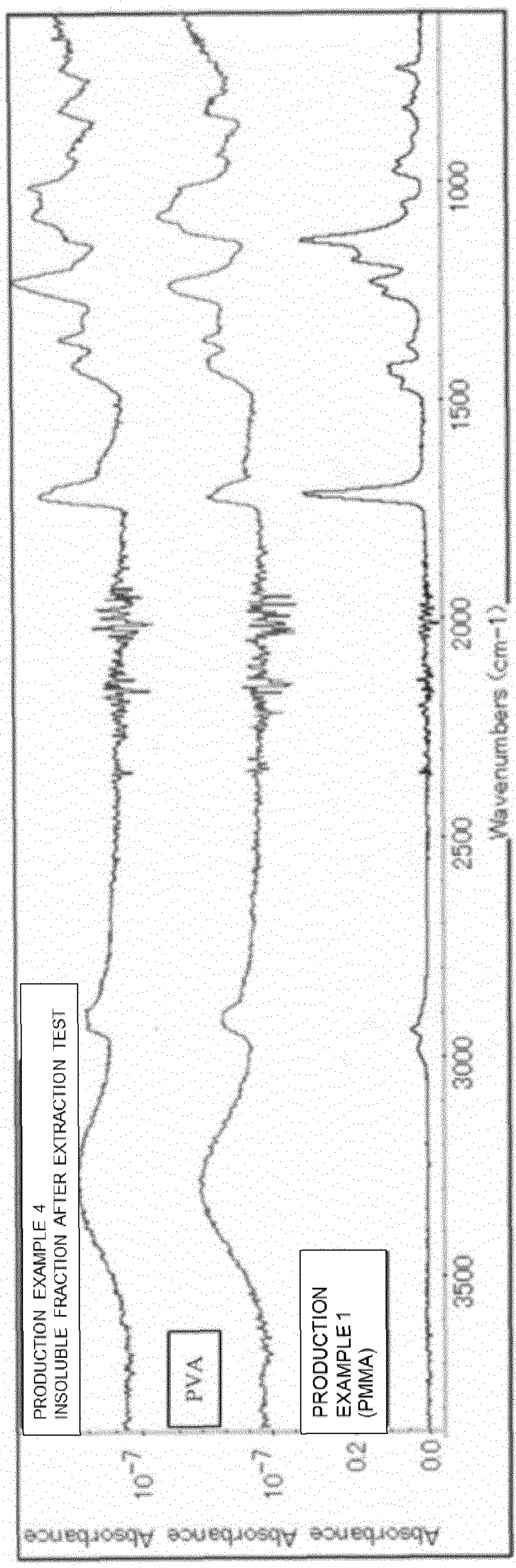
PRODUCTION EXAMPLE 4
INSOLUBLE FRACTION AFTER EXTRACTION TEST
PVA
PRODUCTION
EXAMPLE 1
(PMMA)
Absorbance
Absorbance
Absorbance
10⁻⁷
10⁻⁷
0.2
0.0
3500
3000
2500
2000
1500
1000
Wavenumbers (cm-1)

FIG. 5

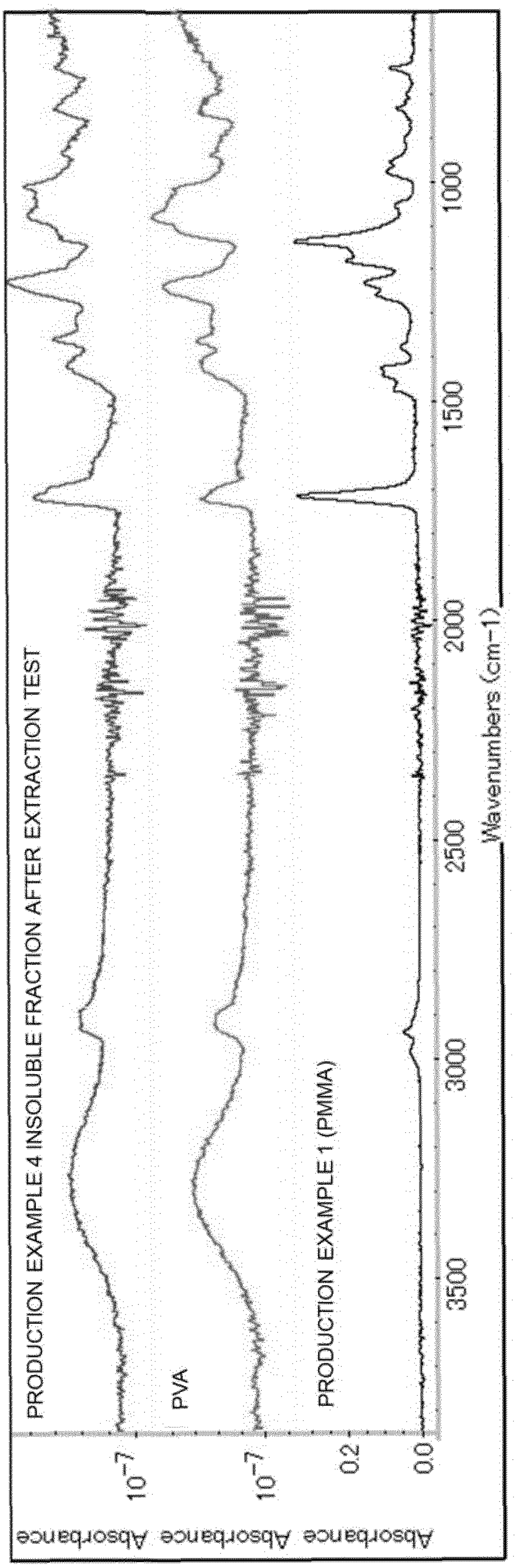
PRODUCTION EXAMPLE 4 INSOLUBLE FRACTION AFTER EXTRACTION TEST
PVA
PRODUCTION EXAMPLE 1 (PMMA)
Absorbance
Absorbance
Absorbance
$10^{-7}$
$10^{-7}$
0.2
0.0
3500
3000
2500
2000
1500
1000
Wavenumbers (cm−1)

FIG. 6

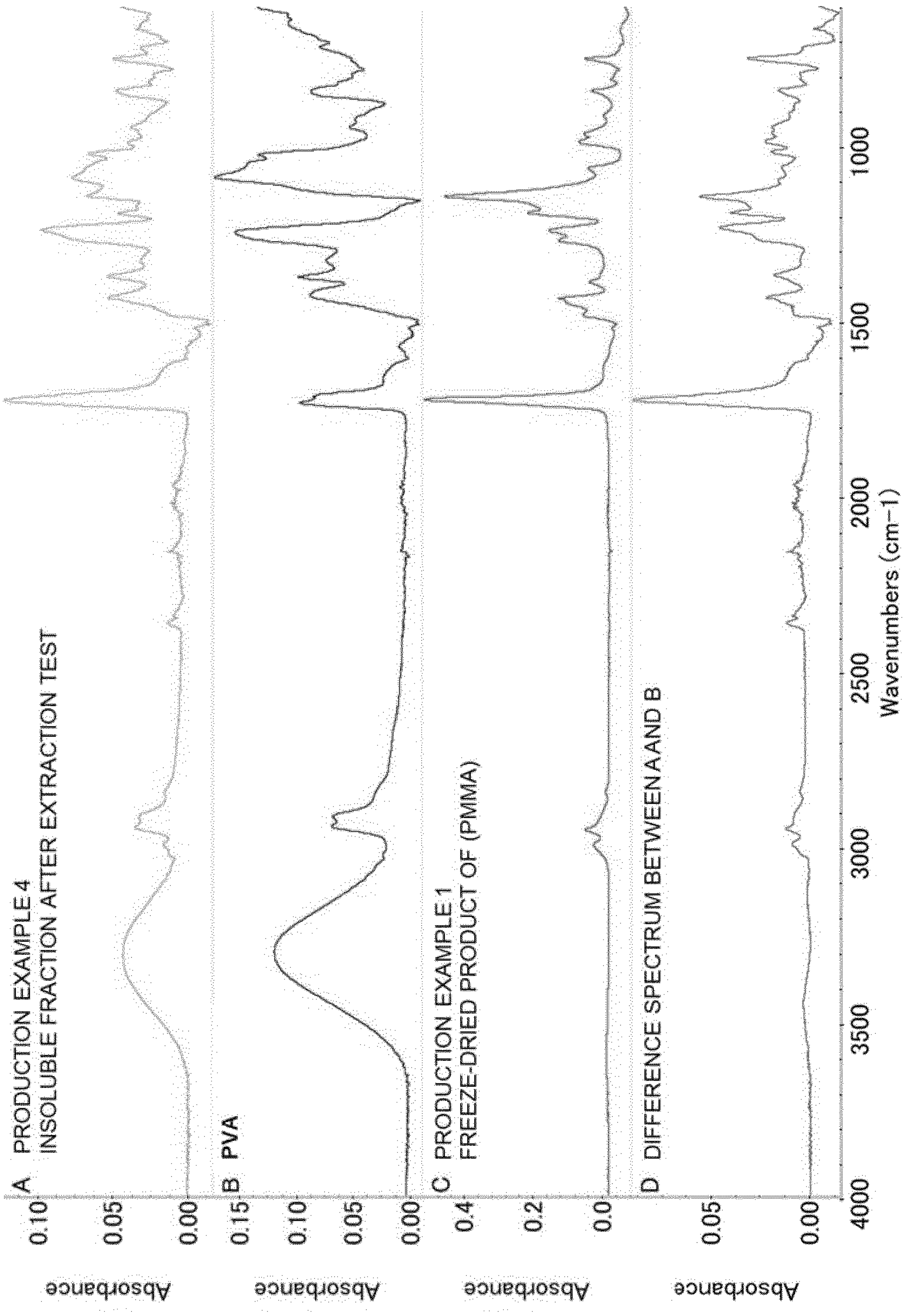
A PRODUCTION EXAMPLE 4
INSOLUBLE FRACTION AFTER EXTRACTION TEST
B PVA
C PRODUCTION EXAMPLE 1
FREEZE-DRIED PRODUCT OF (PMMA)
D DIFFERENCE SPECTRUM BETWEEN A AND B
Absorbance
0.10
0.05
0.00
0.15
0.4
0.2
0.0
4000
3500
3000
2500
2000
1500
1000
Wavenumbers (cm−1)

## INTERNATIONAL SEARCH REPORT

International application No. **PCT/JP2023/021935**

**A. CLASSIFICATION OF SUBJECT MATTER**

***C08L 101/06***(2006.01)i; ***A61K 8/02***(2006.01)i; ***A61K 8/06***(2006.01)i; ***A61K 8/81***(2006.01)i; ***A61Q 19/00***(2006.01)i; ***C08F 216/06***(2006.01)i; ***C08F 257/02***(2006.01)i; ***C08F 265/06***(2006.01)i

FI: C08L101/06; A61K8/02; A61K8/06; A61K8/81; A61Q19/00; C08F216/06; C08F257/02; C08F265/06

According to International Patent Classification (IPC) or to both national classification and IPC

**B. FIELDS SEARCHED**

Minimum documentation searched (classification system followed by classification symbols)

C08L101/06; A61K8/02; A61K8/06; A61K8/81; A61Q19/00; C08F216/06; C08F257/02; C08F265/06

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2021-88554 A (KIRIN HOLDINGS CO., LTD.) 10 June 2021 (2021-06-10) claims, example 4-2 | 1-30 |
| X | JP 2021-88555 A (KIRIN HOLDINGS CO., LTD.) 10 June 2021 (2021-06-10) claims, paragraph [0030], example 4-2 | 1-30 |
| A | JP 2015-506815 A (TAKASAGO INTERNATIONAL CORP.) 05 March 2015 (2015-03-05) claims, example 1 | 1-30 |

☐ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 August 2023** | **29 August 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/021935**

| Patent document cited in search report | Publication date (day/month/year) | Patent family member(s) | Publication date (day/month/year) |
|---|---|---|---|
| JP 2021-88554 A | 10 June 2021 | (Family: none) | |
| JP 2021-88555 A | 10 June 2021 | (Family: none) | |
| JP 2015-506815 A | 05 March 2015 | US 2015/0017214 A1<br>claims, example 1<br>EP 2620211 A2<br>CN 104066500 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022095286 A
- JP 2019202962 A
- JP 2009102216 A
- JP 2017051113 A


### Non-patent literature cited in the description


- *Colloids and Surfaces A: Physicochemical and Engineering Aspects*, 04 August 2014, vol. 56, 139-145
- *Journal of Colloid and Interface Science*, 05 March 2020, vol. 62, 204-212